(19) Europäisches Patentamt

European Patent Office

Office européen des brevets

(11) **EP 4 786 983 A1**

(12) **EUROPEAN PATENT APPLICATION**

(43) Date of publication:
**05.08.2026 Bulletin 2026/32**

(21) Application number: **25155667.6**

(22) Date of filing: **03.02.2025**

(51) International Patent Classification (IPC):
***G01N 33/574*** *(2006.01)*   ***G01N 33/92*** *(2006.01)*
***G16B 40/00*** *(2019.01)*   ***G16H 50/00*** *(2018.01)*

(52) Cooperative Patent Classification (CPC):
**G01N 33/57525; G01N 33/92; G16B 40/00;**
**G16H 10/40; G16H 20/10; G16H 20/40;**
**G16H 50/20; G16H 50/30; G16H 50/70;**
G01N 2800/52

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Ludwig-Maximilians-Universität**
**München, in**
**Vertretung des Freistaates Bayern**
**80539 München (DE)**

(72) Inventors:
• **Mayerle, Julia**
  **80469 München (DE)**
• **Lerch, Markus M.**
  **80331 München (DE)**
• **Mahajan, Ujjwal M.**
  **81549 München (DE)**

(74) Representative: **Altmann Stößel Dick**
**Patentanwälte PartG mbB**
**Theodor-Heuss-Anlage 2**
**68165 Mannheim (DE)**

(54) **PANCREAS CANCER ASSESSMENT METHODS AND DEVICES**

(57)   The present invention relates to a method for assessing pancreatic cancer in a subject, said method comprising (a) determining the biomarkers Ceramide (d18:1;C24:0), Lysophosphatidylethanolamine (C18:0), Phosphatidylethanolamine (C18:0, C22:6), Sphingomyelin (d17:1; C16:0), and CA19.9 in a sample from said subject; (b) comparing the biomarkers determined in step (a) to at least one corresponding reference; and (c) assessing pancreatic cancer in said subject based on said comparing in step (b). The present invention further relates to a computer-implemented training method of training at least one trainable model for assessing pancreatic cancer, to an automated machine learning model, and to a system related thereto.

**Description**

The present invention relates to a method for assessing pancreatic cancer in a subject, said method comprising (a) determining the biomarkers Ceramide (d18:1;C24:0), Lysophosphatidylethanolamine (C18:0), Phosphatidylethanolamine (C18:0, C22:6), Sphingomyelin (d17:1; C16:0), and CA19.9 in a sample from said subject; (b) comparing the biomarkers determined in step (a) to at least one corresponding reference; and (c) assessing pancreatic cancer in said subject based on said comparing in step (b). The present invention further relates to a computer-implemented training method of training at least one trainable model for assessing pancreatic cancer, to an automated machine learning model, and to a system related thereto.

Pancreatic ductal adenocarcinoma (PDAC) is currently the third - and projected to be the second - leading cause of cancer deaths. In the US, its incidence rises by 1.1% annually and it accounts for 18.7% of all new gastrointestinal cancer cases. Five-year survival of all stages has been reported to be 13% and mortality has marginal improvement in recent decades. Only 15% of PDAC cases are diagnosed as localized cancers, but these patients have a 5-year survival rate of 44%, highlighting that early diagnosis is key to improved survival. Balancing prevalence and cumulative risk of PDAC is indicative that a cohort of patients with an incidence increased over the average population, e.g. between 0.5 and 1%, such as chronic pancreatitis (CP, PDAC incidence: 0.49-1.04% (Kim et al., Sci Rep 2023;13(1): 106)), new onset diabetes (NOD, PDAC incidence: 0.5-1% (Singhi et al., Gastroenterology 2019;156(7):2024-40)) and familial pancreatic cancer (3 or more first-degree relatives first-degree relatives with PDAC: 16%-40%, 2 first-degree relatives with PDAC: up to 12%, 1 first-degree relative with PDAC: up to 6% cumulative risk (Llach et al., Cancer Manag Res 2020;12:743-58)) populations might profit from surveillance. These arguments call for the development of a non-invasive test to diagnose PDAC early in at-risk cohorts.

Plasma metabolic signatures predicting PDAC were proposed earlier (Mayerle et al., Gut 2018;67(1):128-37; Pepe et al., J Natl Cancer Inst 2008;100(20):1432-8; Mahajan et al., Gastroenterology 2022;163(5):1407-22). In particular Mahajan et al. (2022) reported high AUC values for the proposed diagnostic method, however required a pre-allocation of patients according to CA19.9 expression. Further known methods for diagnosing pancreatic cancer are summarized in Table 1.

Table 1: Test methods for diagnosing pancreatic cancer

| Test Method | Reference |
| --- | --- |
| Genomic signature; Oral microbiome; antibiotic resistance genes (ARGs) in the oral microbiome | Shen et al. 2024 |
| Protein markers (TIMP1, LRG1) plus cell-free DNA (cfDNA) | Ben-Ami et al. 2023 |
| Protein markers (PIGR, vWF, and Fibrinogen) plus CA19.9 | Byeon et al. 2024 |
| N-glycan signature | Wen et al. 2024 |
| 5-circRNA Panel | Xu et al. 2024 |
| SNP-based biomarker signature (CD44 and CHI3L2) | Seeger et al. 2024 |
| Nanoparticles (Gold NEB) | Digiacomo et al. 2023 |
| Tumor-associated autoantibodies (TAAbs) | Li et al. 2023 |
| Methylation signature of ctDNA (PDACatch) | Wu et al. 2022 |
| 8-Plex biomarker signature plus CA19.9 (Immray Pan-Can-d) | Brand et al. 2022; Katona et al. 2023 |
| Exosome-based transcriptomic signature (miRNAs) | Nakamura et al. 2022 |
| 2-metabolite model (Isoleucine and Adrenic acid) | Cao et al. 2022 |
| CA19-9 to Bilirubin ratio | Boyd et al. 2023 |
| Metabolite markers LC/ESI-MS | Iwano et al. 2021 |
| Bead-based ELISA test (LRG1 and TIMP1) | Fahrmann et al. 2021 |
| Multiplex immunoassays (HE4 and CEA) | Song et al. 2021 |
| Multi-marker panel | Kim et al. 2021 |
| 8 plasma protein biomarkers | Yu et al. 2021 |
| Urine Metabolomic signature | Sahni et al. 2021 |

(continued)

| Test Method | Reference |
|---|---|
| Multianalyte Serum Biomarker Panel | Firpo et al. 2023 |
| Multi-protein biomarker signature plus CA19.9 | Haab et al. 2024 |

Nonetheless, there is still a need for improved biomarkers and algorithms to reliably diagnose pancreatic cancer to improve survival. This problem is solved by the means and methods of the present invention, with the features of the independent claims. Preferred embodiments, which might be realized in an isolated fashion or in any arbitrary combination are listed in the dependent claims.

In accordance, the present invention relates to method for assessing pancreatic cancer in a subject, said method comprising

(a) determining the biomarkers Ceramide (d18:1;C24:0), Lysophosphatidylethanolamine (C18:0), Phosphatidylethanolamine (C18:0, C22:6), Sphingomyelin (d17:1; C16:0), and CA19.9 in a sample from said subject;
(b) comparing the biomarkers determined in step (a) to at least one corresponding reference; and
(c) assessing pancreatic cancer in said subject based on said comparing in step (b).

In general, terms used herein are to be given their ordinary and customary meaning to a person of ordinary skill in the art and, unless indicated otherwise, are not to be limited to a special or customized meaning. As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements. Also, as is understood by the skilled person, the expressions "comprising a" and "comprising an" preferably refer to "comprising one or more", i.e. are equivalent to "comprising at least one". In accordance, expressions relating to one item of a plurality, unless otherwise indicated, preferably relate to at least one such item, more preferably a plurality thereof; thus, e.g. identifying "a cell" relates to identifying at least one cell, preferably to identifying a multitude of cells.

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment" or similar expressions are intended to be optional features, without any restriction regarding further embodiments of the invention, without any restrictions regarding the scope of the invention and without any restriction regarding the possibility of combining the features introduced in such way with other optional or non-optional features of the invention.

The methods specified herein below, preferably, are in vitro methods. The method steps may, in principle, be performed in any arbitrary sequence deemed suitable by the skilled person, but preferably are performed in the indicated sequence; also, one or more, preferably all, of said steps may be assisted or performed by automated equipment. Moreover, the methods may comprise steps in addition to those explicitly mentioned above. Also, the methods may be comprised in other methods; e.g. the method for assessing pancreatic cancer in a subject may be comprised in a method of monitoring a subject at risk of suffering from pancreatic cancer. Also, the methods are preferably at least partially, more preferably fully, computer-implemented, preferably as described in more detail herein below.

As used herein, if not otherwise indicated, the term "about" relates to the indicated value with the commonly accepted technical precision in the relevant field, preferably relates to the indicated value ± 20%, more preferably ± 10%, most preferably ± 5%. Further, the term "essentially" indicates that deviations having influence on the indicated result or use are absent, i.e. potential deviations do not cause the indicated result to deviate by more than ± 20%, more preferably ± 10%, most preferably ± 5%. Thus, "consisting essentially of" means including the components specified but excluding other components except for materials present as impurities, unavoidable materials present as a result of processes used to provide the components, and components added for a purpose other than achieving the technical effect of the invention. For example, a composition defined using the phrase "consisting essentially of" encompasses any known acceptable additive, excipient, diluent, carrier, and the like. Preferably, a composition consisting essentially of a set of components will comprise less than 5% by weight, more preferably less than 3% by weight, even more preferably less than 1% by weight, most preferably less than 0.1% by weight of non-specified component(s). As referred to herein, parameters and coefficients are preferably used with a number of significant digits deemed appropriate by the skilled person, and not necessarily the number of significant digits provided herein. Thus, e.g. a coefficient of 1.968635 provided for CA19.9 herein below, may be used as a coefficient 2, as 2.0, as 1.97, 1.969, and the like, as deemed appropriate by the skilled person. E.g., in case a %probability of a diagnosis is calculated, it may be sufficient to use values of coefficients only to the $2^{nd}$ significant digit, e.g. a value of 2.0 for CA19.9 in the aforesaid example.

The term "fragment" of a biological or chemical molecule, e.g. of a biomarker as specified herein, is used herein in a wide sense relating to any sub-part of the respective molecule comprising the indicated sequence, structure and/or function. Thus, the term includes sub-parts generated by actual fragmentation of a molecule, but also sub-parts derived from the respective molecule in an abstract manner, e.g. in silico. Thus, as used herein, e.g. an ion generated from a biological or chemical molecule, e.g. before or during a mass spectrometry (MS) measurement, may be referred to as a fragment of the parental molecule. Unless specifically indicated otherwise herein, the compounds specified, e.g. may be covalently or non-covalently linked to further atoms, molecules, and/or molecule complexes.

The term "assessing", as used herein, refers to establishing information about the status of the indicated disease or condition, in particular its severity, symptoms, localization, prognosis and/or other relevant information. Said assessing preferably is an aid in diagnosing the indicated disease; as the skilled person will understand, establishing a diagnosis may be based on the aforesaid assessment, however, preferably is based on the aforesaid assessment in combination with further diagnostic information, such as anamnesis data, general physical, mental examination findings, and/or additional metabolic data. Thus, assessing pancreatic cancer may relate to assessing whether a subject suffers from pancreatic cancer, is at risk of suffering from pancreatic cancer, exhibits a medical condition which deteriorates with respect to pancreatic cancer, to establishing the disease stage of pancreatic cancer of a subject, and/or establishing a prognosis with respect to pancreatic cancer. Accordingly, assessing as used herein includes diagnosing pancreatic cancer, predicting the risk for developing pancreatic cancer, and/or predicting any deterioration of the health condition of the subject, in particular, with respect to signs and symptoms accompanying pancreatic cancer. Assessment referred to herein may also be the assessment of a risk of developing pancreatic cancer. In a further embodiment, assessment may be the prediction of the risk that the subject's (health) condition of the subject will deteriorate. Moreover, it will be understood that if the risk of developing pancreatic cancer or risk of the deterioration of the health condition is predicted, typically, the prediction is made within a predictive window. More typically, said predictive window is of from 1 day to 5 years, in a further embodiment of from one week to 2 years.

As referred to herein, assessing pancreatic cancer preferably may also refer to establishing information about differentiating pancreatic cancer from a non-pancreatic cancer disorder as specified herein below, i.e. said assessing preferably is an aid in differentially diagnosing the indicated disease; as the skilled person will understand, establishing a differential diagnosis may be based on the aforesaid assessment, however, preferably is based on the aforesaid assessment in combination with further diagnostic information, preferably as specified herein above. Thus, assessing pancreatic cancer may relate to assessing whether a subject suffers from pancreatic cancer or from a non-pancreatic cancer disorder. Accordingly, assessing as used herein includes classifying pancreatic cancer from benign pancreatic disease as specified herein below. Preferably, in particular in case of differentiating pancreatic cancer from non-pancreatic cancer disorders, the subject was diagnosed to show at least one symptom indicative of pancreatic cancer and/or of a non-pancreatic cancer disorder, preferably as specified herein below. Thus, the method of assessing pancreatic cancer may in particular be applied to a sample of a subject from a pre-selected subgroup of subjects requiring or profiting from differential diagnosis, preferably as specified herein below.

Assessing as referred to herein may relate to a rule-in assessment, i.e. to identifying a subject as belonging to a group of subjects sharing a common feature, e.g. suffering from pancreatic cancer. Assessing, however, may also relate to a rule-out assessment, i.e. to identifying a subject as not belonging to a group of subjects sharing a common feature, e.g. as not suffering from pancreatic cancer. Thus, the assessment may aid in establishing a diagnosis; the assessment may, however, also aid in excluding a diagnosis. Thus, the method as specified may in particular be comprised in a method of monitoring pancreatic cancer. The above applies mutatis mutandis to differential diagnosis, i.e. the differential diagnosis may be a rule-in assessment, i.e. to identifying a subject as suffering from pancreatic cancer or as suffering from a non-pancreatic cancer disorder; it may, however, may also relate to a rule-out assessment, i.e. to identifying a subject as not suffering from pancreatic cancer or as not suffering from a non-pancreatic cancer disorder. Thus, the assessment may aid in establishing a differential diagnosis. As the skilled person is aware of, e.g. excluding pancreatic cancer in a subject showing symptoms thereof may be of help to decide on the further course of treatment.

In view of the above, assessing pancreatic cancer in particular may include or be aiding in diagnosis of pancreatic cancer; in an embodiment to be applied in a specialist or tertiary care setting, in particular with access to a laboratory environment where automated assays can be run; and/or an aid in the diagnosis and assessment of the severity of pancreatic cancer in subjects with signs and symptoms of pancreatic cancer, in an embodiment in conjunction with other laboratory findings and clinical assessments. Preferably, assessing comprises excluding pancreatic cancer, preferably comprises identifying a subject not suffering from pancreatic cancer; comprises diagnosing pancreatic cancer, preferably comprises diagnosing PDAC; and/or comprises staging pancreatic cancer. More preferably, assessing comprises differentiating between pancreatic cancer and non-pancreatic cancer disorders as specified elsewhere herein, preferably pancreatitis, more preferably chronic pancreatitis, Intraductal Papillary Mucinous Neoplasia (IPMNs) and pancreatic cystic lesions (CPL). Even more preferably assessing is classifying pancreas cancer. Nonetheless, in an embodiment, the non-pancreatic cancer disorders do not comprise chronic pancreatitis; thus, in an embodiment, assessing does not comprise differentiating between pancreatic cancer and pancreatitis, preferably chronic pancreatitis.

As will be understood by those skilled in the art, the assessment made in accordance with the present invention, although usually preferred to be, may not be correct for 100% of the investigated subjects. However, the term typically requires that a statistically significant portion of subjects can be correctly assessed. Whether a portion is statistically significant can be determined without further ado by the person skilled in the art using various well known statistic evaluation tools, e.g., determination of confidence intervals, p-value determination, Student's t-test, Mann-Whitney test, etc. Details may be found in Dowdy and Wearden, Statistics for Research, John Wiley & Sons, New York 1983. Typically envisaged confidence intervals are at least 50%, at least 60%, at least 70%, at least 80%, at least 90%, or at least 95%. The p-values are, typically, 0.1, 0.05, or 0.01.

The terms "pancreatic cancer" and "pancreas cancer", as used herein, relate equally to malignant neoplasms which are derived from pancreas cells and, preferably, from pancreatic epithelial cells. Thus, preferably, pancreatic cancer as used herein is pancreatic adenocarcinoma, more preferably pancreatic ductal adenocarcinoma (PDAC). Preferably, the pancreatic cancer is a resectable pancreatic cancer, i.e., preferably, is a pancreatic cancer at a tumor stage permitting, preferably complete, resection of the tumor from the subject. More preferably, said pancreatic cancer is a pancreatic cancer of tumor stage IA-IIB. The symptoms accompanying pancreatic cancer are well known from standard text books of medicine and may include severe abdominal pain, lower back pain, weight loss, new onset diabetes, and jaundice.

The term "non-pancreatic cancer disorder" as used herein, relates to any non-malignant disease of the pancreas. Preferably, the non-pancreatic cancer disorder has at least one symptom which is also a symptom of pancreatic cancer, e.g. presence of at least one pancreatic lesion in an affected subject, jaundice, HbA1c > 6.5%, weight loss in last 6 months, back pain in last weeks, exocrine insufficiency, and/or severe abdominal pain. Preferably, the non-pancreatic cancer disorder has formation of at least one pancreatic cystic lesion as a symptom, wherein said cystic lesion preferably is detectable by an imaging technique such as radiography, computer tomography, magnetic resonance tomography, sonography, or the like. Thus, preferably, a non-pancreatic cancer disorder is pancreatitis, in particular chronic pancreatitis, intraductal papillary mucinous neoplasm (IPMN), cystic pancreatic lesions (CPL), and pancreatic metastases of extrapancreatic origin.

The term "subject", as used herein, relates to an animal, preferably to a mammal. More preferably, the subject is a primate and, most preferably, a human. Preferably, the subject is an apparently healthy subject. Preferably, the subject is a subject at risk of suffering from pancreatic cancer. Risk factors for developing pancreatic cancer are known in the art, e.g. from Brand et al., Gut. 2007;56: 1460-9; or Del Chiaro et al., World J Gastroenterol 2014; 20:12118-12131 and include new-onset diabetes, genetic factors, chronic disease, and age; more preferably, the risk factor is new-onset diabetes. Thus, preferably, the subject is at risk of suffering from pancreatic cancer, i.e., preferably, the subject is a subject from a population with a prevalence of pancreatic cancer of at least 0.5%, preferably at least 1%, more preferably at least 5%, even more preferably at least 10%, most preferably at least 20%. Corresponding populations are known in the art and include in particular populations having a genetic predisposition, preferably familiar pancreatic cancer, including Peutz-Jeghers Syndrome, BRCA1 positivity, or a genetic predisposition for developing pancreatitis; thus, the subject may be from a familial PDAC kinship. Also preferably, the subject at risk of suffering from pancreatic cancer is a subject at least 40 years old, more preferably, at least 50 years old. More preferably, the subject at risk of suffering from pancreatic cancer is a subject suffering from chronic pancreatitis and/or from pancreatic lesions, wherein said pancreatic lesions preferably have been confirmed by an imaging methods such as CT or MRT; thus, the subject preferably has been identified, e.g. by computer tomography (CT), to have pancreatic lesions necessitating further diagnostic assessment. Most preferably, the subject at risk of suffering from pancreatic cancer is a subject with new-onset diabetes, preferably new-onset diabetes type II. Preferably, the subject is a subject in whom new-onset diabetes was diagnosed at most three years, more preferably at most two years, most preferably at most one year, before the method as specified herein is performed on a sample of said subject. Thus, the method preferably is a method for diagnosing pancreatic cancer in a subject suffering from new-onset diabetes.

Also preferably, the subject is a subject in need of differential diagnosis of pancreatic cancer. Thus, the subject preferably is a subject showing at least one symptom of pancreatic cancer and/or of a non-pancreatic cancer disorder, preferably as specified herein above. Preferably, the subject is known or suspected to be suffering from pancreatic cancer and/or is known or suspected to suffer from a non-pancreatic cancer disorder. The skilled person selects the appropriate assessment for a particular subject dependent on the subject's symptoms and existing diagnoses, including tentative diagnoses.

The term "sample", as used herein, refers to a biological sample from a body fluid, preferably blood, plasma, serum, saliva or urine, or a sample derived from cells, tissues or organs, in particular from the pancreas, e.g., by biopsy. Preferably, the sample is a blood, plasma or serum sample, more preferably a serum or plasma sample. Even more preferably, the sample is a blood or plasma sample or is a serum or plasma sample, most preferably, is a plasma sample.

Preferably, the sample is a citrate plasma sample, a heparin plasma sample, or an EDTA plasma sample.

Biological samples can be derived from a subject by techniques known in the art. For example, blood samples may be obtained by blood taking, while tissue or organ samples are to be obtained, e.g., by biopsy. In an embodiment, the sample is known or suspected to comprise biomarkers referred to herein. The aforementioned samples may be pre-treated before they are used for according to the present invention. Said pre-treatment may include treatments required to release or separate the biomarker(s) and/or the analyte(s) or to remove excessive material or waste. Suitable techniques comprise centrifugation, extraction, fractioning, ultrafiltration, protein precipitation followed by filtration and purification and/or enrichment of compounds. Moreover, other pre-treatments may be carried out in order to provide the biomarker and/or analyte in a form or concentration suitable for the intended determination. Suitable and necessary pre-treatments depend on the means used for carrying out the method of the invention and are well known to the person skilled in the art. Pre-treated samples as described before are also comprised by the term "sample" as used in accordance with the present invention.

Preferably, the sample is a fasting sample, in particular a fasting blood, plasma or serum sample. Thus, preferably, the sample is obtained from a fasting subject. A fasting subject, in particular, is a subject who refrained from food and beverages, except for water, prior to obtaining the sample to be tested. Preferably, a fasting subject refrained from food and beverages, except for water, for at least eight hours prior to obtaining the sample to be tested. More preferably, the sample has been obtained from the subject after an overnight fast. Preferably said fasting continued up to at least one hour before sample taking, more preferably up to at least 30 min before sample taking, still more preferable up to at least 15 min before sample taking, most preferably until the sample was taken. Preferably the sample is known or suspected to comprise biomarkers referred to herein. Also preferably, the concentration of CA19.9 in the sample is known or suspected to be less than 37 U/ml, preferably less than 10 U/ml.

The term "biomarker", as used herein, refers to a molecular species which serves as an indicator for a disease or physiological state as referred to herein. Said molecular species can be a chemical compound which is detectable in a sample of a subject, in particular a metabolite of the subject's metabolism. Moreover, the biomarker may also be a molecular species which is derived from said metabolite. In such a case, the actual metabolite will be chemically modified in the sample or during the determination process and, as a result of said modification, a chemically different molecular species, i.e. the analyte, will be the determined molecular species. Also, in case the biomarker has an activity, e.g. a catalytic activity and/or an activating activity, e.g. on target cells, the biomarker may also be determined via said activity, e.g. in an enzymatic assay. It is to be understood that in the aforesaid cases, the analyte may represent the actual biomarker and has the same potential as an indicator for the respective medical condition as the biomarker would have. Preferred modes of determination and analytes for the biomarkers of the present description are described in the context of the respective biomarkers herein below. Moreover, as is understood by the skilled person, a biomarker according to the present invention need not necessarily correspond to one molecular species. Rather, the biomarker may comprise stereoisomers or enantiomers of a compound and/or, e.g. in case the biomarker is a polypeptide, may comprise variant molecular species, e.g. translated from splice variants, glycosylation variants, peptidase processing variants, and the like. In an embodiment, the variants share at least one determinable feature, e.g. an epitope or an activity.

The biomarkers referred to herein and methods for their determination are in principle known in the art: Ceramide (d18:1;C24:0): CAS NO: 102917-80-6, Lysophosphatidylethanolamine (C18:0): CAS NO: 899443-67-5, Phosphatidylethanolamine (C18:0, C22:6): CAS NO: 202647-82-3, Sphingomyelin (d17:1; C16:0): CAS NO: 123065-40-7, and CA19.9: CAS NO: 92448-22-1. Further preferred biomarkers are Histidine (CAS NO: 71-00-1), Proline (CAS NO:147-85-3), Tryptophan (CAS NO: 73-22-3), Ceramide (d18:2,C24:0): CAS NO: 135941-18-3, Lysophosphatidylethanolamine (C18:2): CAS NO: 85046-18-0, Sphingomyelin (35:1): CHEBI:133629, Sphingomyelin (41:2): CHEBI:85762, and Sphingomyelin (d18:2,C17:0): SpectraBase Compound ID: D4yNg15DQf3. Preferably, at least one additional biomarker is determined in addition to the aforesaid biomarkers. Preferably, said additional biomarker is selected from the list consisting of aspartate aminotransferase (EC 2.6.1.1), alanine aminotransferase (EC 2.6.1.2), platelet count, haptoglobin (e.g. Genbank Acc No. NP_001119574.1), alpha2-macroglobulin (e.g. Genbank Acc No. NP_000005.3), apolipoprotein A1 (e.g. Genbank Acc No. NP_000030.1), bilirubin (CAS NO: 635-65-4), cholesterol (CAS NO: 57-88-5), hyaluronan (CAS NO: 9004-61-9), prothrombin index, hepatocyte growth factor (HGF, e.g. Genbank Acc No. NP_000592.3), and urea (CAS NO: 57-13-6).

The term "determining" as used herein refers to semi quantitative or quantitative determination of a biomarker referred to herein. Determining the amount of a biomarker may be carried out by any technique which allows for establishing a measure of quantity of a biomarker in a semi quantitative or quantitative manner. Suitable techniques depend on the molecular nature and the properties of the biomarkers and are discussed elsewhere herein in more detail.

In principle, the amount of a biomarker can be determined by determining a complex of the analyte with a detection compound, in particular an antibody or fragment thereof, i.e. in an immunoassay. Said determining of a complex of the analyte may be performed in any format deemed appropriate by the skilled person, in particular a sandwich, competition, or other assay format. Said assays will develop a signal which is indicative for the amount of a biomarker. The amount of a biomarker may in an embodiment be determined in an activity assay, in particular in case the biomarker has catalytic, e.g. enzymatic, or signaling activity.

Preferably, the amount of a biomarker may be determined by detecting the amount of molecular species of the biomarker, or of fragments thereof. E.g., small molecule biomarkers may be detected as such or as their ions in mass spectrometry (MS). For polypeptide biomarkers detection of fragments thereof may be technically easier to put into practice. However, also other methods for detecting the amount of molecular species of the analyte are available, including chromatographic separation techniques such as liquid chromatography (LC), high performance liquid chromatography (HPLC), gas chromatography (GC), thin layer chromatography, and/or size exclusion or affinity chromatography, coupled to appropriate detection devices. Such a detection device may e.g. be a photometer, e.g. an UV/VIS-photometer or an MS device. Appropriate devices and methods are known in the art. Further suitable methods comprise measuring a physical or chemical property specific for the biomarker such as its precise molecular mass or an NMR spectrum. Said methods comprise, preferably, biosensors, optical devices coupled to immunoassays, biochips, analytical devices such as mass-spectrometers, NMR-analyzers, surface plasmon resonance measurement equipment or chromatography devices.

The biomarkers to be determined in accordance with the present invention are as such known in the art. Moreover, methods for the determination of the amount of the biomarkers are known to the skilled person as well. For example, the biomarkers can be measured as described in the Examples section.

More preferably, determining of at least one biomarker comprises mass spectrometry (MS). For mass spectrometry, the analytes in the sample are ionized in order to generate charged molecules or molecule fragments. Afterwards, the mass-to-charge of the ionized analyte, in particular of the ionized biomarkers, or fragments thereof is measured. Thus, the mass spectrometry step preferably comprises an ionization step in which the biomarkers to be determined are ionized. Of course, other compounds present in the sample/eluate are ionized as well. Ionization of the biomarkers can be carried out by any method deemed appropriate, in particular by electron impact ionization, fast atom bombardment, electrospray ionization (ESI), atmospheric pressure chemical ionization (APCI), matrix assisted laser desorption ionization (MALDI). More preferably, the ionization step (for mass spectrometry) is carried out by electrospray ionization (ESI). Accordingly, the mass spectrometry is preferably ESI-MS (or if tandem MS is carried out, ESI-MS/MS).

Mass spectrometry as used herein encompasses all techniques which allow for the determination of the molecular weight (i.e. the mass) or a mass variable corresponding to a compound, i.e. a biomarker, to be determined in accordance with the methods proposed herein. Preferably, a combination of mass spectrometry with a separation and/or enrichment method is used, in particular gas chromatography mass spectrometry (GC-MS), liquid chromatography mass spectrometry (LC-MS), direct infusion mass spectrometry or Fourier transform ion-cyclotrone-resonance mass spectrometry (FT-ICR-MS), capillary electrophoresis mass spectrometry (CE-MS), high-performance liquid chromatography coupled mass spectrometry (HPLC-MS), quadrupole mass spectrometry, any sequentially coupled mass spectrometry, such as MS-MS or MS-MS-MS, inductively coupled plasma mass spectrometry (ICP-MS), pyrolysis mass spectrometry (Py-MS), ion mobility mass spectrometry or time of flight mass spectrometry (TOF). How to apply these techniques is well known to the person skilled in the art. Moreover, suitable devices are commercially available. More preferably, mass spectrometry as used herein relates to LC-MS and/or GC-MS, i.e. to mass spectrometry being operatively linked to a prior chromatographic separation step. More preferably, mass spectrometry as used herein encompasses quadrupole MS.

The term "reference", as used herein, relates to a value, e.g. an amount or any value derived therefrom, e.g. a score, which can be correlated to a medical condition and, preferably, which allows for the assessment as referred to herein to be made, more preferably enables allocation of a subject into either a group of subjects suffering from a disease or condition or being at risk for developing it, or a group of subjects which do not suffer from said disease or condition or which are not at risk for developing it. Such a reference can be a threshold value, e.g. a threshold amount, which separates these groups from each other. Accordingly, the reference may be a value which allows for allocation of a subject into a group of subjects suffering from a disease or condition or being at risk for developing it, or not. For example, the reference may be a value which allows for allocation of a subject into a group of subjects suffering from pancreatic cancer, or being at risk of developing pancreatic cancer. The reference may, however, also be a reference range, e.g., in an embodiment, a range of values for which pancreatic cancer can be excluded. Furthermore, the reference may be a value calculated from the aforesaid values, e.g. from the amounts of two or more biomarkers, in an embodiment to provide a score, e.g. a predictor score as specified elsewhere herein. A suitable reference separating the two groups can be provided without further ado e.g. by the statistical tests referred to herein elsewhere based on values of biomarkers from suitable reference groups as specified herein below. As the skilled person understands, it may not always be possible, although particularly envisaged, to provide a reference unambiguously allocating each and every possible value of a biomarker to one of the aforesaid groups; thus, there may be a range of values for which a clear assessment cannot be provided. Preferably, however, as indicated above, a reference enables the assessment to be made for each and every value of a biomarker or set of biomarkers which may be measured. As the skilled person understands, the specific value of a reference may depend on the assessment intended and on parameters thereof; thus, the reference value for assessing pancreatic cancer may typically be different from the reference value for assessing e.g. chronic pancreatitis. Relevant parameters having an influence on the reference may in particular be sensitivity and specificity of assessment.

A reference may in particular be derived from at least one reference group, the term "reference group" relating to a group of subjects with known status with regard to the assessment. Thus the reference group may e.g. be a group of subjects for which it is known whether they suffer from pancreatic cancer. The population of subjects in a reference group preferably comprises a plurality of subjects, e.g. at least 5, 10, 50, 100, 1,000, or 10,000 subjects. Typically, the subject to be diagnosed and the subjects of the said reference group are of the same species. The reference applicable for an individual subject may vary depending on various physiological parameters such as age, gender, or subpopulation. As is understood by the skilled person, prevalence of pancreatic cancer in the population is low; thus, a reference may be derived also from the average population. Assuming that contribution of actually afflicted subjects is low, such an average population reference group may be treated as a reference group known not to suffer from pancreatic cancer; preferably, in such case, the size of the reference group is sufficiently high, e.g. at least 100, more preferably at least 1000, even more preferably at least 10000 subjects. In view of the description herein, the skilled person understands that a reference group may, in principle, also be a mixed population of subjects with regard to pancreatic cancer, provided that the status of each member of said mixed population with regards to pancreatic cancer is or becomes known before deriving a reference from such group.

Reference amounts can, in principle, be calculated for a cohort of subjects based on the average or mean values for a given parameter such as biomarker amount by applying standard statistically methods. In particular, accuracy of a test such as a method aiming to diagnose an event, or not, is best described by its receiver-operating characteristics (ROC) (see especially Zweig 1993, Clin. Chem. 39:561-577). The ROC graph is a plot of all of the sensitivity/specificity pairs resulting from continuously varying the decision threshold over the entire range of data observed. The clinical performance of a diagnostic method depends on its accuracy, i.e. its ability to correctly allocate subjects to a certain prognosis or diagnosis. The ROC plot indicates the overlap between the two distributions by plotting the sensitivity versus 1-specificity for the complete range of thresholds suitable for making a distinction. On the y-axis is sensitivity, or the true-positive fraction, which is defined as the ratio of number of true-positive test results to the product of number of true-positive and number of false-negative test results. This has also been referred to as positivity in the presence of a disease or condition. It is calculated solely from the affected subgroup. On the x-axis is the false-positive fraction, or 1-specificity, which is defined as the ratio of number of false-positive results to the product of number of true-negative and number of false-positive results. It is an index of specificity and is calculated entirely from the unaffected subgroup. Because the true- and false-positive fractions are calculated entirely separately, by using the test results from two different subgroups, the ROC plot is independent of the prevalence of the event in the cohort. Each point on the ROC plot represents a sensitivity/-specificity pair corresponding to a particular decision threshold (i.e. reference). A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. If the ROC plot falls completely below the 45° diagonal, this is easily remedied by reversing the criterion for "positivity" from "greater than" to "less than" or vice versa. Qualitatively, the closer the plot is to the upper left corner, the higher the overall accuracy of the test. Dependent on a desired confidence interval, a threshold can be derived from the ROC curve allowing for the diagnosis or prediction for a given event with a proper balance of sensitivity and specificity, respectively. Accordingly, the reference to be used for the aforementioned method of the present invention, i.e. a threshold which allows to discriminate between subjects being at risk and not being at risk can be generated, usually, by establishing a ROC for said cohort as described above and deriving a threshold amount therefrom. Dependent on a desired sensitivity and specificity for a diagnostic method, the ROC plot allows deriving suitable thresholds. It will be understood that an optimal sensitivity may be desired for excluding a subject for being at increased risk (i.e. a rule-out), whereas an optimal specificity may be envisaged for a subject to be assessed as being at an increased risk (i.e. a rule-in).

The term "comparing" as used herein encompasses comparing the determined amount for a biomarker as referred to herein to a reference. It is to be understood that comparing as used herein refers to any kind of comparison made between the value for the amount with the reference. However, it is to be understood that preferably identical types of values are compared with each other, e.g., if an absolute amount is determined, the reference shall also be an absolute amount, if a relative amount is determined, the reference shall also be a relative amount, etc. The aforesaid comparison of identical types of values is also referred to a comparing to a "corresponding" value, e.g. a corresponding reference, herein. The term comparing also encompasses comparing a calculated score with a suitable reference core. Thus, preferably, the corresponding reference is a value of the quantitative parameter allowing the determination of step (c) to be made. Also preferably, the corresponding reference comprises references for each biomarker derived from at least one subject known to suffer from pancreatic cancer. More preferably, the at least one corresponding reference comprises references for each biomarker derived from at least one subject known to not suffer from pancreatic cancer. Most preferably, the reference is a reference predictor score obtained using a trainable model trained as described elsewhere herein.

The comparison may be carried out manually or computer assisted. The value of the amount and the reference can be, e.g., compared to each other and the said comparison can be automatically carried out by a computer program executing an algorithm for the comparison. The computer program carrying out the said evaluation will provide the desired assessment in a suitable output format. As set forth above, it is also envisaged to calculate a score based on the amounts of the biomarkers, which may also be referred to as "predictor score", in particular a single score, and to compare this score to a reference score. The calculated score in an embodiment combines information on the amounts of the biomarkers. Moreover, in the score, the biomarkers may be weighted in accordance with their contribution to the establishment of the differentiation, wherein the weighting factor of the individual biomarkers may be different. The score can be regarded as a classifier parameter for the assessing as set forth herein. In particular, it enables providing the assessment based on a single score. Thus, the skilled person does not have to interpret the entire information on the amounts of the individual biomarkers. Using a scoring system as described herein, values of different dimensions or units for the biomarkers may be used since the values will be mathematically transformed into the score. Accordingly, e.g. values for absolute concentrations may be combined in a score with peak area ratios and/or enzymatic activity values. The reference score to be applied may be elected based on the desired sensitivity and/or the desired specificity. Preferably, the reference, in particular the reference score, is obtained by training at least one trainable model for assessing pancreas cancer in a subject, as described herein below.

Preferably, comparing comprises calculating a predictor score according to formula (I):

$$P = \frac{1}{1 + e^{-z}}$$

wherein P= predictor score; and z = $\beta_0 + \beta_1 X_1 + \beta_2 X_2 + \cdots + \beta_n X_n$
wherein coefficients $\beta_0$, $\beta_1$, $\beta_2$, ..., $\beta_2$ are preferably selected from Table 2 or from Table 3, and wherein variables $X_1$, $X_2$, ..., $X_n$ are measurement values of the biomarkers, wherein said measurement values are preferably measurement values indicated as µg/ml and/or are log10 transformed and z-score normalized according to standard methods known to the skilled person. Also preferably, to address biases related to inter-platform variations and internal standards in the quantification of biomarkers, covariate shift correction using linear regression on matched samples is preferably applied. Thus, preferably, for each biomarker, a linear regression model based on the matched training and test samples is provided and correction slope and intercept are provided, followed by adjusting the test dataset according to the derived linear model to maintain consistency across different platforms. Effectiveness of the aforesaid correction may be evaluate e.g. by calculating a Concordance Correlation Coefficient (CCC), which measures the agreement between the corrected test data and the training data.

Preferably, the aforesaid coefficients are used with at least one significant digit, more preferably with at least two significant digits, still more preferably with at least three significant digits, most preferably with all significant digits as provided in Table 2 and/or Table 3.
The method comprises step (a) determining the biomarkers Ceramide (d18:1;C24:0), Lysophosphatidylethanolamine (C18:0), Phosphatidylethanolamine (C18:0, C22:6), Sphingomyelin (d17:1; C16:0), and CA19.9 in a sample from a subject. The biomarkers and methods for determining them have been described herein above.
Preferably, the method comprises further determining at least one, preferably at least two, more preferably at least three, even more preferably at least four, most preferably all, marker(s) selected from the list consisting of Histidine, Proline, Tryptophan, Ceramide (d18:2,C24:0), Lysophosphatidylethanolamine (C18:2), Sphingomyelin (35:1), Sphingomyelin (41:2), and Sphingomyelin (d18:2,C17:0), preferably in step (a). Also preferably, the method further comprises determining at least one further biomarker, preferably selected from the list consisting of aspartate aminotransferase, alanine aminotransferase, platelet count, haptoglobin, alpha2-macroglobulin, apolipoprotein A1, bilirubin, cholesterol, hyaluronan, prothrombin index, hepatocyte growth factor (HGF), Tissue inhibitor of metalloproteinases (TIMP), and urea, preferably in step (a). Also preferably, the method comprises further diagnostic steps, preferably sonography, magnetic resonance imaging, radiography, transient elastography, and/or determining subject age and/or gender, preferably in, preceding, or following step (a).
The method further comprises step (b) comparing the biomarkers determined in step (a) to at least one corresponding reference. Suitable references and methods of comparing have described herein above. Preferably, step (b) comprises calculating a predictor score for assessing pancreatic cancer from at least two, at least three, more preferably at least four, most preferably all, of the biomarkers referred to herein. Thus, step (b) may in particular comprise calculating a predictor score from the biomarkers Ceramide (d18:1;C24:0), Lysophosphatidylethanolamine (C18:0), Phosphatidylethanolamine (C18:0, C22:6), Sphingomyelin (d17:1; C16:0), and CA19.9, preferably as specified herein above.
The term "comparing" has been specified herein above. In certain embodiments, comparing may comprise a comparison to an implied reference; e.g. in case a predictor score is calculated as a probability of a subject suffering from pancreatic cancer, an express reference value may not be required, since the meaning of a probability of e.g. 0.9, and/or a %probabilty of e.g. 90% of a subject to suffer from pancreatic cancer are self-evident for the skilled person.

Preferably, the method is at least partially computer-implemented and at least said comparing in step (b) is performed by a trained automated machine learning derived generalized logistic regression model, wherein said model preferably was trained as specified herein below.

The method further comprises step (c) assessing pancreatic cancer in said subject based on said comparing in step (b). The term "assessing" has been specified herein above.

Advantageously, it was found in the work underlying the present invention that the method of assessing pancreas cancer as described herein allows for improved assessment of pancreatic cancer. In particular, the biomarker combinations (signatures) presented herein are a cost-effective tools designed to achieve a very high NPV, with very high specificity to safely exclude PDAC in patients with, CP or pancreatic lesions which require further diagnostic assessment. The signatures significantly outperform CA19.9 alone and thus can serve as an adjunct screening tool in higher-risk cohorts and is planned for the exclusion of PDAC in new-onset diabetes patients.

The definitions made above apply mutatis mutandis to the following. Additional definitions and explanations made further below also apply for all embodiments described in this specification mutatis mutandis.

The present invention also relates to a method for assessing and treating pancreatic cancer, said method comprising the steps of the method for assessing pancreatic cancer as specified herein above and the further step of treating said pancreatic cancer in a subject identified to suffer therefrom.

Methods for treating pancreatic cancer are, in principle, known in the art and include in particular surgery and chemotherapy.

The present invention further relates to a computer-implemented training method of training at least one trainable model for assessing pancreas cancer in a subject, the method comprising:

(i) providing the trainable model;
(ii) retrieving labeled training subject data comprising biomarker data of subjects having known pancreas cancer states, wherein said biomarker data are data of the biomarkers Ceramide (d18:1;C24:0), Lysophosphatidylethanolamine (C18:0), Phosphatidylethanolamine (C18:0, C22:6), Sphingomyelin (d17:1; C16:0), and CA19.9; and
(iii) training the trainable model on the labeled training subject data,

wherein said trainable model is a Generalized Linear Model (GLM).

The term "training", as used herein, relates to a process of determining parameters of at least one machine learning and/or deep learning model, specifically of the algorithm of the machine learning and/or deep learning model, specifically on at least one training data set or set of training data. The training specifically may comprise at least one optimization or tuning process, wherein a best parameter combination, e.g. according to at least one optimization procedure, is determined.

The term "trainable model", as used herein, relates to at least one mathematical model configured for transforming one or more input values into one or more output values by using one or more parameters which may be adjusted in order to enable the model to be trained. The trainable model specifically may be a trainable mathematical model which is trainable on at least one training data set using one or more of machine learning, deep learning, neural networks, or other form of artificial intelligence. The term specifically may refer, without limitation, to the fact that the trainable model can be further trained, optimized or updated based on additional training data. Specifically, the trainable model is trained on a training dataset. The trainable model may be trained by using machine learning. The trainable model may be at least partially data-driven by being trained on data from historical training data.

The trainable model specifically may comprise at least one trainable model selected from the group consisting of a decision tree model, specifically at least one of an XGBoost model and a Random Forest model; a Support Vector Machine (SVM) model, specifically, a linear kernel SVM; a nearest neighbors model, specifically a KNeighbors Classifier; a Bayes model, specifically a GaussianNB() model; a regression model, specifically at least one of a LogisticRegression; a linear and a nonlinear regression model, e.g. a regression model comprising transformed features, such as log-transformed or polynomial; an Artificial Neural Network (ANN), specifically a non-linear Artificial Neural Network (ANN), in particular a deep learning architecture such as Convolutional NN, Recurrent NN, Long Short Term Memory NN, and the like; a kernel-based method; a tree regression model; a distributed gradient-boosting framework model, specifically a light gradient-boosting machine model (LightGBM classifier). As referred to herein, the trainable model is a Generalized Linear Model (GLM), preferaly a binomial GLM.

Consequently, the term "trained model", as used herein, relates to a trainable model which has gone through at least one training process as defined above, by applying, at least once, a set of training data to the trainable model. Specifically, one or more parameters of the trainable model might have been adapted, on the basis of the training data, in order to transform the trainable model into a trained model. Specifically, the trained model may be a trainable model which was trained on at least one training dataset, also denoted training data. The trained model may be or may comprise a classifier, configured for classifying an object, on the basis of one or more input variables, describing the object. The trained model may comprise at least one trained model selected from the group of trainable models as specified herein above.

The trainable model and/or the trained model specifically may be a model taken from a software library, providing a plurality of trainable models. As an example, the Scikit-learn open-source software library may be mentioned, for trainable models and/or trained models being programmed in the Python programming language. Additionally or alternatively, also, as an example, reference may be made to the eXtreme Gradient Boosting (XGBoost) open-source software library, specifically providing gradient boosting trainable models, being programmed in one or more of the programming languages C++, Java, Python, R, Julia, Perl and Scala. Other libraries, however, are also feasible, as well as customized trainable models not retrieved from software libraries.

The term "classify", as used herein, relates to the process of assigning an object to one or more predetermined or determinable classes. The classifying specifically may comprise a prediction or assignment providing an indication to which class out of a plurality of classes an object belongs, e.g. in accordance with one or more predetermined properties of the object.

The term "pancreas cancer state", as used herein, relates to at least one item of information indicating at least one physical state or health state of a subject with respect to pancreas cancer. Thus, a pancreas cancer state may e.g. be "pancreas cancer: yes" or pancreas cancer:no", may be "afflicted by pancreas cancer", or the like. As referred to herein, indication of a non-cancer state, e.g. "chronic pancreatitis:yes", may also be a pancreas cancer state, in as far as it implies that the subject does not suffer from pancreas cancer.

As outlined above, the trainable model is configured for classifying a subject's health condition into a pancreas cancer state. Thus, as discussed above, the trainable model specifically may assign a subject's health condition to a state of suffering from pancreatic cancer, or not.

The term "retrieve", as used herein, relates to the process of obtaining data from a data source. The data source may vary, in accordance with the specific application. Thus, in the context of the present invention, the retrieving of the labeled training subject data may take place by at least one of the following: downloading the labeled training subject data from at least one data source, such as from at least one data storage device, from a web- or cloud-based data storage device; obtaining the labeled training subject data via at least one computer network, such as the Internet; obtaining the labeled training subject data via at least one wire-based and/or wireless interface. The retrieving of the training data may fully or partially take place automatically, such as by automatic download of the labeled training subject data from publications of data on clinical studies, and/or may fully or partially take manually. Semiautomatic retrieving processes are also possible. Thus, as an example, data of clinical studies may be downloaded, e.g. automatically, and the data may be processed in order to generate the labelled training subject data, e.g. by manual labeling of the training subject data downloaded from a web-based data source and/or by other data cleaning or data processing steps. Additionally or alternatively, the retrieving of the labeled training subject data may also comprise the actual measurement of the data, e.g., by evaluating subject samples, preferably as specified herein elsewhere.

The term "labeled", as used herein, relates to the property of training data having, besides the actual biomarker data, additional information indicating a classification of the data. Thus, as an example, the labeled training subject data may contain, specifically for each subject or each data set of a subject, besides the biomarker data, an indication of the known pancreas cancer state of the subject. Thus, as an example, for each subject or each data set of the subject, besides the biomarker data of their respective subject, information may be comprised, indicating the pancreas cancer state of the subject. Additionally, the following state may also be comprised: the subject being in an apparently healthy state. The pancreas cancer state of the respective subjects may be obtained by other diagnostic means, such as by detecting one or more of the above-mentioned symptoms of the respective subjects contributing to the training subject data.

Consequently, the labeled training subject data may be obtained by using data from a plurality of subjects, such as by using data from one or more clinical studies. As an example, data from at least 100 subjects, specifically data from at least 500 subjects or more, may be used for generating the labeled training subject data. For, each subject, firstly, biomarker data may be generated. Further, for each subject, information on the pancreas cancer state of the respective subject may be obtained. As outlined above, diagnostic indicators other than biomarker data are preferably used for obtaining the pancreas cancer state of the respective subject, such as by detecting the presence or absence of the characteristic symptoms for each of the pancreas cancer states. Then, for each subject, the biomarker data are labeled at least with the respective information on the pancreas cancer state of the respective patient, in order to obtain a labeled training data set for the respective subject. In addition, and optionally, additional information on the respective subject may be comprised in the labeling, such as information on the age of the subject, information on the gender of the subject, medical information on the subject and the like, preferably as specified herein above. The aggregate of the labeled training data set of the subjects may then form part of the labeled training subject data.

Preferably, the labeled training data may also be obtained by using data from one or more medical studies. Also, preferably, the training data are those of Table 4a, optionally further including the training data of Table 4b herein below.

The term "biomarker data", as used herein, relates to all data pertaining to amount or concentrations of biomarkers in a sample of a subject. Preferably, the biomarker data are obtained as specified herein elsewhere. The biomarker data preferably comprise biomarker data from a sample as specified herein above.

One or more than one trainable model may be used. Even though the method generally relates to trainable models suited for classifying the patient's health condition, the trainable model specifically may be or may comprise at least one trainable SVM model and/or at least one trainable XGBoost model. These models, for the present application, out of the available trainable model is in standard software libraries, have shown to provide excellent classification results for classifying a patient's health condition according to pancreas cancer states.

As outlined above, the label of the training data specifically may contain information on the respective pancreas cancer state. Thus, generally, the labelling of the training subject data to obtain the labeled training subject data may contain information on the respective pancreas cancer state, as shown e.g. in Table 4a. The labeling may be part of the method or may be part of a preceding step of generating the training data, e.g. during one or more clinical studies.

The predetermined pancreas cancer states may comprise or may consist of: the patient suffering from pancreas cancer; the patient not suffering from pancreas cancer and/or the patient suffering from a benign pancreatic disease. Thus, the trainable model, when being in a trained state, may be configured for assessing patients suspected to suffer from a pancreas cancer state. Besides the categories listed above, one or more additional states may be comprised by the predetermined group of the pancreas cancer states, in particular as specified herein above; e.g. the subject suffering from pancreatic adenocarcinoma and/or the subject suffering from pancreatic ductal adenocarcinoma (PDAC) and/or the subject being in an apparently healthy state and/or the subject suffering from pancreatitis.

The biomarker data of the labeled training subject data in step (ii) preferably are derived from a blood or blood-derived sample and are, preferably, obtained by MS analysis, more preferably by LC-MS.

The training method further may comprise at least one hyperparameter tuning step for tuning hyperparameters of the trainable model. The term "hyperparameter tuning step", as used herein, relates to the process of adjusting, specifically optimizing, one or more hyperparameters of the trainable model. Thus, trainable models, e.g. trainable models from standard software libraries such as Scikit-learn, typically require one or more hyperparameters to be predetermined before starting the training process. The hyperparameter tuning step may comprise the pre-adjusting of these hyperparameters, e.g. in order to provide a suitable convergence for the training process. As an example, the labeled training data or at least a part thereof may be used for an initial test for training the trainable model, trying different sets of hyperparameters, wherein the performance of the training process is evaluated on the basis of measurable training performance values such as the speed of convergence, and, wherein, the set of hyperparameters providing a superior performance may finally be chosen for the trainable model. The trainable model may then be trained with this specific set of hyperparameters.

The hyperparameter tuning step, specifically, may be performed at least once before performing step (iii). It is, however, also possible to implement a plurality of tuning steps, intermitting with one or more partial training steps, e.g. in order to improve or even optimize the tuning of the hyperparameters during the process.

As outlined above, the trainable model specifically may comprise or specifically may consist of a trainable GLM model. As also outlined in further detail below, the trainable GLM model specifically may be trained using the following hyperparameters:

Regularization: Ridge; Logistic regression link: logit; Number of iterations: 40, Nlambad = 30, Lambda.max = 32.331; Sort_metric = "F1", i.e. preferably the F1 score is calculated from the harmonic mean of the precision and recall; Stopping tolerance = 3, i.e. preferably if the performance of iteration is not improved after 3 subsequent rounds.

As will be outlined in further detail below, this set of hyperparameters is suited to provide training results leading to a trained GLM model on the basis of the labeled training data, the trained GLM model being capable of classifying the patient's health condition with high reliability. For further details on these hyperparameters, as an example, reference may be made to the Scikit-learn library.

The training subject data, as discussed above, specifically may be obtained from one or more patient studies. Specifically, the labeled training subject data may be patient data obtained from a plurality of different studies. More specifically, the labeled training subject data may contain patient data from differing age groups. The label of the training subject data, as outlined above, may contain information on the study. Additionally or alternatively, the label of the training subject data may contain information on the patient, such as on the group the respective patient belongs to. More specifically, the label may contain age information, such as information on the respective age group of the patients. Thus, the labelling of the training subject data to obtain the labeled training data may additionally contain information on the respective age groups of the patients.

The training method specifically may comprise splitting the labeled training subject data into a training data set and a test data set. Thus, more specifically, the splitting into a training data set and a test data set may comprise x%-y% splitting of the labeled training subject data, with x% being in the range 60 to 90, specifically in the range 65 to 75, and more specifically x%=70, and with y%=100-x. Therein, x% denotes the training data set and y% denotes the test data set.

The training method may further compromise a cross-validation in the training data set. Thus, the data of the training data set may be further split into k subsets or folds of equal size, with k being at least 2 or more folds, specifically in the range 3 to 10, more specifically k=5. The training and validation may be repeated k times, and each time the data of a different subset or fold may be used for validation. Additionally or alternatively, the training method may further comprise repeating the splitting of the labeled training data into the training data set and the test data set one or more times, specifically for a number of times in the range of 10 to 40 times, more specifically 20 times. Thus, a validation of the model may be performed with an x1%-y1% splitting of the labeled training subject data, and at least one additional x2%-y2% splitting of the labeled training subject data, with x1 and x2 describing different partitions of the labeled training subject data, wherein, specifically, x1%=x2%. As an example, a degree of discrepancy between the results obtained for the multiple iterations may be used for qualifying and/or quantifying the training result.

Additionally or alternatively, the training method may further comprise repeatedly performing step (iii) with multiple splittings. The multiple splittings may comprise an x1%-y1% splitting of the labeled training subject data and at least one additional x2%-y2% splitting of the labeled training subject data, with x1 and x2 describing different partitions of the labeled training subject data, wherein, specifically, x1%=x2%. Alternatively or additionally, the multiple splittings may comprise differing splittings of the labeled training data, specifically with x1%≠x2%. Again, optionally, the training results, e.g. at least one numeric value qualifying and/or quantifying the training results, e.g. qualifying and/or quantifying the accuracy of the training, may be compared.

As outlined above, the training method specifically may comprise splitting the labeled training subject data into a training data set and a test data set. The training method may, then, further comprises the following step:
at least one validation step, the validation step comprising testing the accuracy of the training by using the test data set.

The testing of the accuracy specifically may comprise using at least one model accuracy metrics. Various model accuracy metrics are generally known in the field of machine learning. Specifically, one or more or even all of the following model accuracy metrics may be used: overall accuracy, balanced accuracy, weighted f1 score. Other options, however, are also feasible.

The training method may further comprise the following step:
determining at least one target set of coefficients $\beta_0$, $\beta_1$, ..., $\beta_n$ as specified herein above, by using at least one feature extraction method. Again, various feature extraction methods are generally known to the skilled person in the field of machine learning.

In a further aspect of the present invention, a computer-implemented method of assessing pancreas cancer in a subject is disclosed. The computer-implemented method comprises the following method steps, which preferably are performed in the given order. However, a different order is also feasible. Further, it is possible to perform two or more of the method steps simultaneously or in a fashion overlapping in time. Further, it is also possible to perform one, more than one or even all of the method steps repeatedly.

The computer-implemented method for assessing pancreas cancer comprises the following steps:

(A) retrieving at least one trained model, preferably as specified herein above;
(B) retrieving biomarker data of the subject; and
(C) applying the trained model to the biomarker data, thereby assessing pancreas cancer in the subj ect.

The trained model specifically may be trained or have been trained by using the training method according to the present invention, such as according to any one of the embodiments described above and/or according to any one of the embodiments described in further detail below. Thus, for most of the terms, definitions and options as described above or as described in further detail below apply mutatis mutandis.

As outlined in further detail above, the computer-implemented method specifically may assign the patient or the patient's health condition to one of the pancreas cancer states of the predetermined group of pancreas cancer states. The subject preferably is a subject as specified herein above. The group of predetermined pancreas cancer states may be the same as discussed above in the context of the training method. The biomarker data preferably comprise data for biomarkers also determined during the training of the trained model. Thus, as discussed above, the trained model may be trained by using the training method described herein.

The present invention also relates to a system comprising (i) a measuring device for determining the biomarkers Ceramide (d18:1;C24:0), Lysophosphatidylethanolamine (C18:0), Phosphatidylethanolamine (C18:0, C22:6), Sphingomyelin (d17:1; C16:0), and CA19.9; and (ii) an evaluation device operably linked to the measuring device, said evaluation device comprising a data processor comprising instructions for carrying out a comparison of the biomarkers determined by the measuring device to at least one corresponding reference.

The term "system", as used herein, relates to a collection of the indicated means which are operatively linked to each other. Said means may be implemented in a single system or may be physically separated devices which are operatively linked to each other. Preferably, the system further comprises an output device outputting the result of the assessment performed by the evaluation device. Preferably, the system further comprises a data storage device configured for storing, in particular in electronic form, data provided by the devices of the system, data provided by user input, in particular one or more reference, and/or instructions for performing the assessment, in particular machine-readable instructions for performing the assessment as specified herein above. As is understood by the skilled person, said data storage device(s) may be part of the measuring device and/or the evaluation device, and/or may be standalone data storage device(s) operatively linked to the other devices of the system.

The term "measuring device", as referred to herein, includes each and every device allowing determining at least one, preferably at least two, more preferably at least three, even more preferably at least four, most preferably all biomarkers referred to herein. Preferably, the measuring device comprises a receptacle for a sample. The receptacle may directly contact the sample, or may be a receptacle for a further means receiving the sample, wherein the further means may be e.g. a multi-well plate, to which a sample or a multiplicity of samples may be applied. Preferably, the measuring device comprises at least one detector for determining at least one biomarker, e.g., an MS device, a biosensor, a solid support coupled to a ligand specifically recognizing a biomarker, a Plasmon surface resonance devices, an NMR spectrometer, and the like).

The term "evaluation device", as referred to herein, may be any means capable of providing the analysis as specified; preferably, the evaluation device is or comprises a data processing means, in particular at least one processor, such as a microprocessor, a handheld device such as a mobile phone, or a computer. How to link the means in an operating manner will depend on the type of means included into the device. Preferably, the means are comprised by a single device. Preferably, the instructions and interpretations are comprised in an executable program code comprised in the device, such that, as a result of determination, an assessment of pancreatic cancer may be output to a user. Typical devices are those which can be applied without the particular knowledge of a specialized technician.

Preferably, the system comprises at least one processor, the processor being configured, e.g. by software programming, for performing the training method and/or the computer-implemented method for assessing pancreas cancer according to the present invention. More preferably, the system is system for assessing pancreas cancer and comprises at least one processor, the processor being configured, e.g. by software programming, for performing the method for assessing pancreas cancer, preferably the computer-implemented method for assessing pancreas cancer, according to the present invention.

The term "processor", as used herein, relates to an arbitrary logic circuitry configured for performing basic operations of a computer or system, and/or, generally, to a device which is configured for performing calculations or logic operations. In particular, the processing unit may be configured for processing basic instructions that drive the computer or system. As an example, the processor may comprise at least one arithmetic logic unit (ALU), at least one floating-point unit (FPU), such as a math co-processor or a numeric coprocessor, a plurality of registers, specifically registers configured for supplying operands to the ALU and storing results of operations, and a memory, such as an L1 and L2 cache memory. In particular, the processor may be a multi-core processor. Specifically, the processing unit may be or may comprise a central processing unit (CPU). Additionally or alternatively, the processor may be or may comprise a microprocessor, thus specifically the processing unit's elements may be contained in one single integrated circuitry (IC) chip. Additionally or alternatively, the processing unit may be or may comprise one or more application-specific integrated circuits (ASICs) and/or one or more field-programmable gate arrays (FPGAs) or the like. The processing unit specifically may be configured, such as by software programming, for performing one or more evaluation operations.

The present invention also relates to an automated machine learning model obtained or obtainable according to the method according to the method of the present invention, preferably tangibly embedded on a data storage means.

The present invention also relates to a database for assessing pancreas cancer comprising at least one set of biomarker coefficients for calculating a predictor score from quantitative data of the biomarkers Ceramide (d18:1;C24:0), Lysophosphatidylethanolamine (C18:0), Phosphatidylethanolamine (C18:0, C22:6), Sphingomyelin (d17:1; C16:0), and CA19.9 in a sample, and optionally at least one reference predictor score and/or at least one reference predictor range.

In a further aspect of the present invention, a computer program is proposed, the computer program comprising instructions which, when the program is executed by at least one processor of a computer or a computer network cause the processor to perform the training method according to the present invention, such as according to any one of the embodiments of the training method described above and/or according to any one of the embodiments of the training method described in further detail below.

In a further aspect of the present invention, a computer program is proposed, the computer program comprising instructions which, when the program is executed by at least one processor of a computer or a computer network cause the processor to perform the assessment method according to the present invention, such as according to any one of the embodiments of the assessment method described above and/or according to any one of the embodiments of the assessment method described in further detail below.

In a further aspect of the present invention, a computer-readable storage medium is proposed, specifically a non-transient computer-readable storage medium, the computer readable storage medium comprising instructions which, when the instructions are executed by at least one processor of a computer or a computer network cause the processor to perform the training method according to the present invention, such as such as according to any one of the embodiments of the training method described above and/or according to any one of the embodiments of the training method described in further detail below.

In a further aspect of the present invention, a computer-readable storage medium is pro-posed, specifically a non-transient computer-readable storage medium, comprising instructions which, when the instructions are executed by at least one processor of a computer or a computer network cause the processor to perform the assessment method according to the present invention, such as according to any one of the embodiments of the assessment method described above and/or according to any one of the embodiments of the assessment method described in further detail below.

As outlined above, the methods as proposed herein are computer-implemented. As used herein, the terms "computer-readable data carrier" and "computer-readable storage medium" specifically may refer to non-transitory data storage means, such as a hardware storage medium having stored thereon computer-executable instructions. The computer-readable data carrier or storage medium specifically may be or may comprise a storage medium such as a random-access memory (RAM) and/or a read-only memory (ROM).

Thus, specifically, the method steps of the methods as indicated above may be performed by using a computer or a computer network, preferably by using a computer program.

Further disclosed and proposed herein is a computer program product having program code means, in order to perform any one of the methods according to the present invention in one or more of the embodiments enclosed herein when the program is executed on a computer or computer network. Specifically, the program code means may be stored on a computer-readable data carrier and/or on a computer-readable storage medium.

In view of the above, the following embodiments are particularly envisaged:

Embodiment 1: A method for assessing pancreatic cancer in a subject, said method comprising

(a) determining the biomarkers Ceramide (d18:1;C24:0), Lysophosphatidylethanolamine (C18:0), Phosphatidylethanolamine (C18:0, C22:6), Sphingomyelin (d17:1; C16:0), and CA19.9 in a sample from said subject;
(b) comparing the biomarkers determined in step (a) to at least one corresponding reference; and
(c) assessing pancreatic cancer in said subject based on said comparing in step (b).

Embodiment 2: The method of embodiment 1, wherein said pancreatic cancer is pancreatic adenocarcinoma, preferably pancreatic ductal adenocarcinoma (PDAC).

Embodiment 3: The method of embodiment 1 or 2, wherein assessing comprises excluding pancreatic cancer, preferably comprises identifying a subject not suffering from pancreatic cancer.

Embodiment 4: The method of any one of embodiments 1 to 3, wherein said assessing comprises diagnosing pancreatic cancer, preferably comprises diagnosing PDAC.

Embodiment 5: The method of any one of embodiments 1 to 4, wherein said assessing comprises staging pancreatic cancer.

Embodiment 6: The method of any one of embodiments 2 to 5, wherein said assessing comprises prognosticating pancreatic cancer.

Embodiment 7: The method of any one of embodiments 1 to 6, wherein said assessing comprises differentiating between pancreatic cancer and a non-pancreatic cancer disorder, preferably pancreatitis, more preferably chronic pancreatitis.

Embodiment 8: The method of any one of embodiments 1 to 7, wherein said subject is a human.

Embodiment 9: The method of any one of embodiments 1 to 8, wherein said subject is a subject at risk of suffering from pancreatic cancer.

Embodiment 10: The method of any one of embodiments 1 to 9, wherein said subject with an increased risk of suffering from pancreatic cancer is known or suspected to suffer from new onset type 2 diabetes, chronic pancreatitis, pancreatic cystic lesions, and/or is from a familial PDAC kinship.

Embodiment 11: The method of any one of embodiments 1 to 10, wherein said method is comprised in a method of monitoring a subject at risk of suffering from pancreatic cancer.

Embodiment 12: The method of any one of embodiments 1 to 11, wherein said method comprises further determining at least one, preferably at least two, more preferably at least three, even more preferably at least four, most preferably all, marker(s) selected from the list consisting of Histidine, Proline, Tryptophan, Ceramide (d18:2,C24:0), Lysophosphatidylethanolamine (C18:2), Sphingomyelin (35:1), Sphingomyelin (41:2), and Sphingomyelin (d18:2,C17:0), preferably in step (a).

Embodiment 13: The method of any one of embodiments 1 to 12, wherein at least one, preferably each, biomarker is determined by a method comprising independently selected a mass spectrometry (MS), preferably LC/MS, more preferably LC/MS-MS, an enzymatic assay, and/or an immunoassay.

Embodiment 14: The method of any one of embodiments 1 to 13, wherein said sample is a bodily fluid sample, preferably a blood sample or a blood-derived sample, more preferably a blood, plasma, or serum sample, even more preferably a plasma or serum sample. Embodiment 15: The method of any one of embodiments 1 to 14, wherein the concentration of CA19.9 in said sample is known or suspected to be less than 37 U/ml, preferably less than 10 U/ml.

Embodiment 16: The method of any one of embodiments 1 to 15, wherein said determining said biomarkers comprises determining for each of said biomarkers a value of a quantitative parameter correlating with the amount of said biomarker in said sample.

Embodiment 17: The method of any one of embodiments 1 to 16, wherein step (b) comprises calculating a predictor score for assessing pancreatic cancer from all of said biomarkers.

Embodiment 18: The method of embodiment 17, wherein step (b) comprises determining all biomarkers of embodiment 1 and wherein said predictor score is calculated according to formula (I)

$$P = \frac{1}{1 + e^{-z}}$$

wherein P= predictor score; and $z = \beta_0 + \beta_1 X_1 + \beta_2 X_2 + \cdots + \beta_n X_n$
and wherein variables $X_1$, $X_2$, ..., $X_n$ are measurement values of said biomarkers.

Embodiment 19: The method of embodiment 18, wherein coefficients $\beta_0$, $\beta_1$, $B_2$, ..., $\beta_n$ are selected from Table 2 or from Table 3.

Embodiment 20: The method of any one of embodiments 1 to 19, wherein said at least one corresponding reference is a value of said quantitative parameter allowing the determination of step (c) to be made.

Embodiment 21: The method of any one of embodiments 1 to 20, wherein said at least one corresponding reference comprises references for each biomarker derived from at least one subject known to suffer from pancreatic cancer.

Embodiment 22: The method of embodiment 21, wherein amounts for each of the biomarkers being essentially identical or similar to the at least one corresponding reference are indicative for a subject suffering from pancreatic cancer; and/or amounts for each of the biomarkers being different from the corresponding references are indicative for a subject not suffering from pancreatic cancer.

Embodiment 23: The method of any one of embodiments 1 to 22, wherein said at least one corresponding reference comprises references for each biomarker derived from at least one subject known to not suffer from pancreatic cancer.

Embodiment 24: The method of embodiment 23, wherein amounts for each of the biomarkers being essentially identical or similar to the corresponding references are indicative for a subject not suffering from pancreatic cancer; and/or amounts for each of the biomarkers being different from the corresponding references are indicative for a subject suffering from pancreatic cancer.

Embodiment 25: The method of any one of embodiments 1 to 24, wherein said method comprises determining at least one further biomarker, preferably selected from the list consisting of aspartate aminotransferase, alanine aminotransferase, platelet count, haptoglobin, alpha2-macroglobulin, apolipoprotein A1, bilirubin, cholesterol, hyaluronan, prothrombin index, hepatocyte growth factor (HGF), Tissue inhibitor of metalloproteinases (TIMP), and urea.

Embodiment 26: The method of any one of embodiments 1 to 25, wherein said method comprises further diagnostic steps, preferably sonography, magnetic resonance imaging, radiography, transient elastography, and/or determining subject age and/or gender.

Embodiment 27: The method of embodiment 26, wherein said method is computer-implemented.

Embodiment 28: The method of any one of embodiments 1 to 27, wherein said method is computer implemented and wherein said comparing in step (b) is performed by a trained automated machine learning derived generalized logistic regression model.

Embodiment 29: The method of embodiment 28, wherein said model was trained as specified in any one of embodiments 31 to 47.

Embodiment 30: The method of any one of embodiments 1 to 29, wherein said reference is a reference predictor score obtained using a trainable model trained according to the method according to any one of embodiments 31 to 47.

Embodiment 31: A computer-implemented training method of training at least one trainable model for assessing pancreas cancer in a subject, the method comprising:

(i) providing the trainable model;
(ii) retrieving labeled training subject data comprising biomarker data of subjects having known pancreas cancer states, wherein said biomarker data are data of the biomarkers Ceramide (d18:1;C24:0), Lysophosphatidylethanolamine (C18:0), Phosphatidylethanolamine (C18:0, C22:6), Sphingomyelin (d17:1; C16:0), and CA19.9; and
(iii) training the trainable model on the labeled training subject data,

wherein said trainable model is a Generalized Linear Model (GLM).

Embodiment 32: The method of embodiment 31, wherein said assessing is classifying pancreas cancer.

Embodiment 33: The method of embodiment 31 or 32, wherein the trainable model is a binomial GLM.

Embodiment 34: The method of any one of embodiments 31 to 33, wherein the labelling of the training subject data to obtain the labeled training subject data contains information on the respective pancreas cancer state.

Embodiment 35: The method of any one of embodiments 31 to 34, wherein the group of predetermined pancreas cancer states comprises, preferably consists of: the subject suffering from pancreatic adenocarcinoma and/or the subject suffering from pancreatic ductal adenocarcinoma (PDAC).

Embodiment 36: The method of any one of embodiments 31 to 35, wherein the group of predetermined pancreas cancer states further comprises: the subject being in an apparently healthy state and/or the subject suffering from pancreatitis.

Embodiment 37: The method of any one of embodiments 31 to 36, wherein the biomarker data of the labeled training subject data in step (ii) are derived from a bodily fluid sample, preferably a blood sample or a blood-derived sample, more preferably a blood, plasma, or serum sample, even more preferably a plasma or serum sample and are, preferably, obtained by a method comprising independently selected a mass spectrometry (MS), preferably LC/MS, more preferably LC/MS-MS, an enzymatic assay, and/or an immunoassay.

Embodiment 38: The method of any one of embodiments 31 to 37, wherein the trainable GLM model is trained using the following hyperparameters:

Regularization: Ridge;
Logistic regression link: logit;
Number of iterations: 40,
$N_{lambad}$ = 30,
Lambda.max = 32.331
Sort_metric = "F1", and/or
Stopping tolerance = 3.

Embodiment 39: The method of any one of embodiments 31 to 38, wherein the labeled training subject data contain subject data from differing age groups.

Embodiment 40: The method of any one of embodiments 31 to 39, wherein the labelling of the training subject data to obtain the labeled training data additionally contains information on the respective age groups of the subjects.

Embodiment 41: The method of any one of embodiments 31 to 40, wherein the training comprises splitting the labeled training subject data into a training data set and a test data set.

Embodiment 42: The method of embodiment 41, wherein the splitting into a training data set and a test data set comprises a x%-y% splitting of the labeled training subject data, with x% being in the range 60 to 90, specifically in the range 65 to 75, and more specifically x%= 70, and with y%=100-x.

Embodiment 43: The method of any one of embodiments 31 to 42, wherein the method comprises a cross-validation in the training data set.

Embodiment 44: The method of any one of embodiments 31 to 43, wherein the method further comprises at least one validation step, the validation step comprising testing the accuracy of the training by using the test data set.

Embodiment 45: The method of any one of embodiments 31 to 44, wherein testing of the accuracy comprises using at least one model accuracy metrics, specifically at least one model accuracy metrics selected from the group consisting of: area under the curve (AUC), overall accuracy, balanced accuracy, positive predictive value, negative predictive value, true positive rate, true negative rate, false positive rate, false negative rate, and diagnostic odds ratio, preferably AUC.

Embodiment 46: The method of any one of embodiments 31 to 45, wherein said training data comprise the data of Table 4a and, optionally of Table 4b.

Embodiment 47: The method of any one of embodiments 31 to 46, further comprising at least one feature of any one of embodiments 1 to 30.

Embodiment 48: An automated machine learning model obtained or obtainable according to the method according to any one of embodiments 31 to 47, preferably tangibly embedded on a data storage means.

Embodiment 49: A database for assessing pancreas cancer comprising at least one set of biomarker coefficients for calculating a predictor score from quantitative data of the biomarkers Ceramide (d18:1;C24:0), Lysophosphatidylethanolamine (C18:0), Phosphatidylethanolamine (C18:0, C22:6), Sphingomyelin (d17:1; C16:0), and CA19.9 in a sample, and at least one reference predictor score and/or at least one reference predictor range.

Embodiment 50: The database of embodiment 49, wherein said database is tangibly embedded on a data storage means.

Embodiment 51: A computer-implemented method of assessing pancreas cancer in a subject, the method comprising

(A) retrieving at least one trained model, preferably as specified herein above;
(B) retrieving biomarker data of the subject; and
(C) applying the trained model to the biomarker data, thereby assessing pancreas cancer in the subject.

Embodiment 52: A system comprising

(i) a measuring device for determining the biomarkers Ceramide (d18:1;C24:0), Lysophosphatidylethanolamine (C18:0), Phosphatidylethanolamine (C18:0, C22:6), Sphingomyelin (d17:1; C16:0), and CA19.9; and
(ii) an evaluation device operably linked to the measuring device, said evaluation device comprising a data processor comprising instructions for carrying out a comparison of the biomarkers determined by the measuring device to at least one corresponding reference.

Embodiment 53: The system of embodiment 52, wherein the instructions for carrying out a comparison comprise instructions to carry out said comparison according to step (b) of the method according to any one of embodiments 1 to 30.

Embodiment 54: The system of embodiment 52 or 53, adapted to perform a method according to any one of embodiments 1 to 38, preferably comprising tangibly embedded instructions which, when carried out by the data processor, cause the system to perform a method according to any one of embodiments 1 to 38.

Embodiment 55: The system of any one of embodiments 52 to 54, wherein said system further comprises a database according to embodiment 49 or 50 operably coupled to the data processor.

Embodiment 56: The system of any one of embodiments 52 to 55, wherein said detection means is capable of specifically detecting said biomarkers.

Embodiment 57: The system of any one of embodiments 52 to 56, wherein said system is a system for assessing pancreatic cancer in a subject, and wherein said evaluation device further comprises means for assessing pancreatic cancer in said subject based on the comparison.

Embodiment 58: The system of any one of embodiments 52 to 57, wherein said evaluation device is capable of automatically receiving values for the amounts of the biomarkers from the measuring device.

Embodiment 59: A method for assessing and treating pancreatic cancer, said method comprising the steps of the method according to any one of embodiments 1 to 30 and the further step of treating said pancreatic cancer in a subject identified to suffer therefrom.

Embodiment 60: Use of the biomarkers Ceramide (d18:1;C24:0), Lysophosphatidylethanolamine (C18:0), Phosphatidylethanolamine (C18:0, C22:6), Sphingomyelin (d17:1; C16:0), and CA19.9 for assessing pancreatic cancer.

All references cited in this specification are herewith incorporated by reference with respect to their entire disclosure content and the disclosure content specifically mentioned in this specification.

Figure Legends

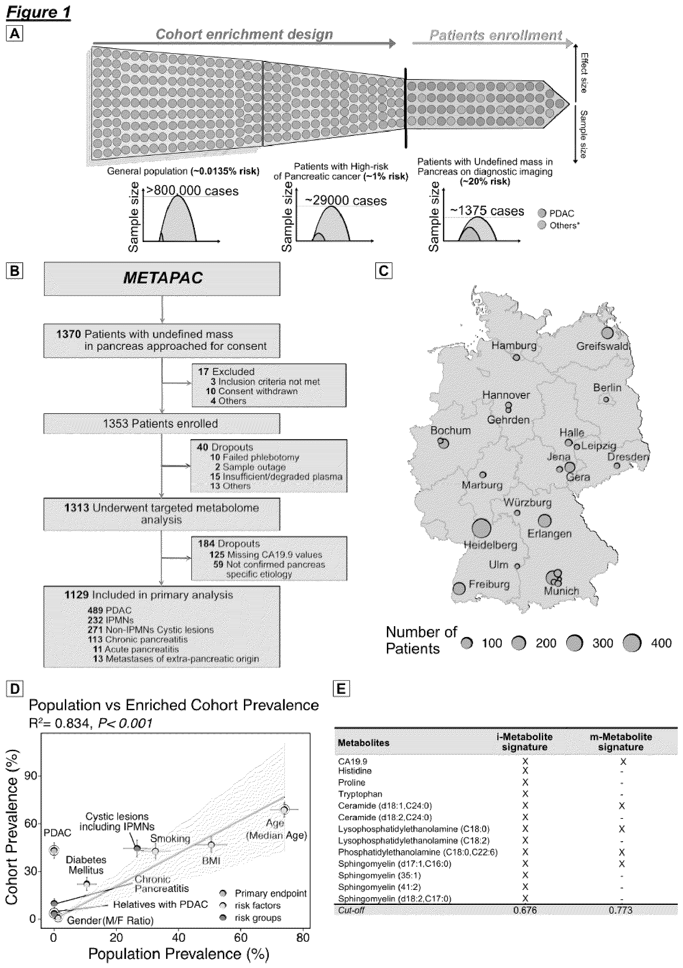

**Figure 1. Trial enrollment and analysis populations. (A)** Graphical explanation of cohort enrichment design: The incidence of PDAC in the general population (0.00014%) increases to 1% among individuals with a genetic predisposition, such as those with familial PDAC (>3 FDR), chronic pancreatitis, or lean NOD patients. A sample size calculation for a prospective diagnostic study within this cohort warrants the recruitment of 29,000 patients. By targeting patients with an undefined pancreatic lesion detected through diagnostic CT-scan increases the effect size, hereby enriching the endpoint of PDAC prevalence in this population to 20%. **(B)** METAPAC study: A total of 1353 participants were enrolled, and 1129 participants were included in the primary analysis. **(C)** Enrollment of number of patients per center across 23 German centers. **(D)** Scatter plot illustrating correlation between population prevalence and cohort prevalence for risk factors (orange) and at-risk groups (green). The encircled points illustrate the comparison of PDAC prevalence within the general population versus the prevalence in an enriched cohort. Of note, Incidence equals prevalence in PDAC, Age is depicted as median values and gender is depicted as male to female ratio. $R^2$: Pearson's correlation coefficient. **(E)** Tabular representation of metabolites involved in i- or *m-Metabolic signature,* with X indicating being part of the signature.

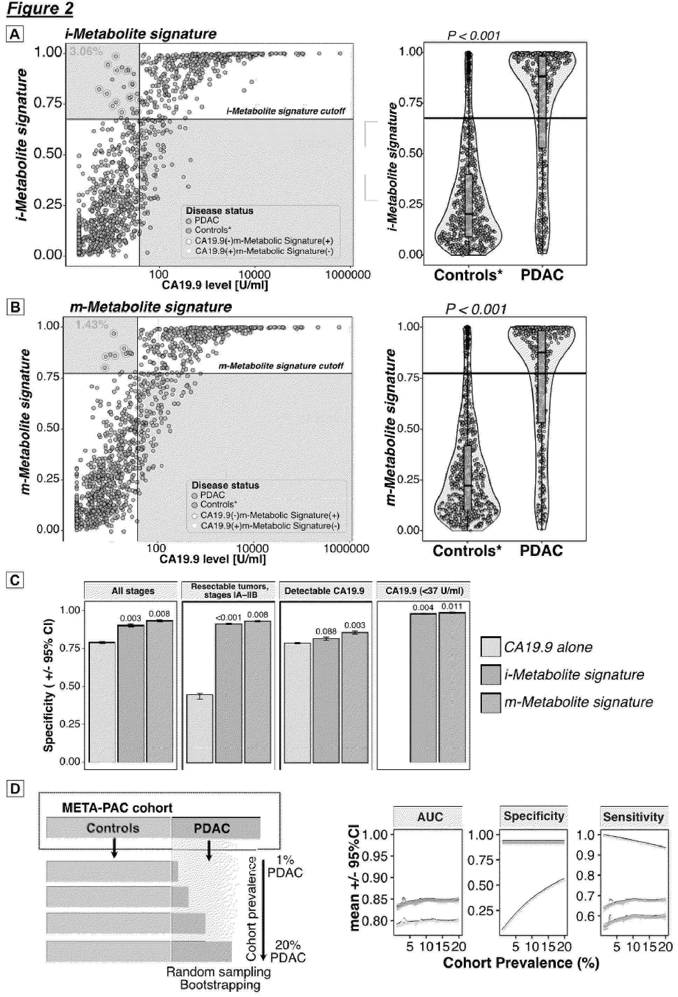

**Figure 2. Prediction score of *i-* and *m-Metabolic signature*. (A-B)** Scatter plot illustrating the *i-Metabolic signature* (A) and *m-Metabolic signature* (B) performance for differential diagnosis of PDAC compared to CA19.9 alone. The y-axis depicts the predictive scores, whereas the x-axis depicts CA19.9 levels. The orange encircled points denote subjects that benefit from the *i-* or *m-Metabolic signature* in predicting PDAC (False Negatives with CA19.9 alone), and blue encircled points represent subjects that benefit by avoiding misdiagnosis (False Positives with CA19.9 alone). Asterisk depicts controls (IPMNs, Non-IPMNs Cystic lesions, Chronic pancreatitis, Acute pancreatitis and Metastases of extra-pancreatic origins) in the cohort. Adjoining boxplot illustrates the distribution of prediction score of *i-* or *m-Metabolic signature* in PDAC vs controls. Student's t-test for PDAC vs controls in boxplot. **(C)** Comparison of specificity of *i-* and *m-Metabolite signature* with CA19.9 alone. Error bars indicate 95% confidence interval. Student's t-test for *i-* or *m-Metabolite signature* vs CA19.9 alone in each subgroup. **(D)** Random Bootstrapping: A cohort prevalence ranging from 1% to 20% was mimicked by randomly selecting PDAC cases, which were compared to controls to assess the diagnostic performance of CA19.9, the *i-* (blue) and *m-Metabolic signature (red)*. Here, the AUC, specificity, and sensitivity for both signatures operated independently of the cohort prevalence, while the specificity of CA19.9 increased, and its sensitivity decreased with increased prevalence. *P*<0.05 considered statistically significant.

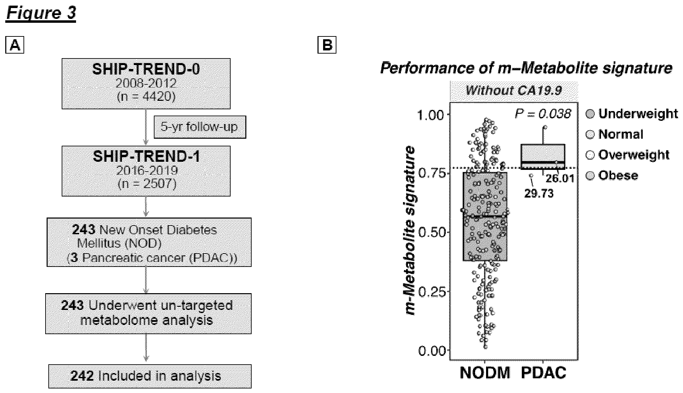

**Figure 3. Prediction score of *m-Metabolic signature* in SHIP-TREND-1. (A)** Patient selection from SHIP-TREND-1. Total 243 out of 2507 participants with NOD were identified and 242 participants were included for further analysis. **(B)** Boxplot illustrating distribution of prediction score of m-Metabolite signature with and without simulated CA19.9 levels. Student's t-test for NOD (without PDAC) vs NOD-PDAC in boxplot. *P*<0.05 considered statistically significant.

The following Examples shall merely illustrate the invention. They shall not be construed, whatsoever, to limit the scope of the invention.

*Example 1: Study design*

METAPAC (Plasma-Metabolome signature for the diagnosis of pancreatic cancer) is a prospective, consecutive, multi-disciplinary, investigator blinded multicentric study, designed to validate the performance of a previously identified plasma metabolite signatures(Mayerle et al. 2018; Mahajan et al. 2022) for early-stage PDAC exclusion in a population at risk. This phase IV study adhered to the EDRN guidelines (Srivastava and Wagner 2020) and was conducted at 23 German academic and community-based institutions. The study adhered to the STARD (Cohen et al. 2016) and TRIPOD guidelines (Collins et al. 2015). The study protocol was approved by the local institutional review board under the reference number BB 079/16 at the University Medicine Greifswald and 736-16 at LMU University. All participants or their legal representatives provided their written informed consent. Clinische Studien Gesellschaft (CSG) mbH, Berlin, Germany collected and monitored the data. The study statistician (first author) analyzed the data and vouches for the completeness and precision of the data and for the fidelity of the study to the protocol, along with the corresponding author.

*Example 2:Study population*

Sample size was calculated based on an expected sensitivity of 88% and specificity of 85%, with a margin of error of 5% and statistical power of 80% ($\alpha$ = 0.05). The PDAC incidence equaling prevalence was expected with 20% in a population with an undefined lesion of the pancreas detected on imaging. Minimum required sample size was determined with 1375 participants. Patients with a undefined pancreatic lesions identified through CT imaging who required further diagnostic evaluation were included. The exclusion criteria were set to be inability to give informed consent, current pregnancy and conditions precluding surgery, biopsy or clinical follow-up.

*Example 3: Clinical procedures*

Eligible participants with informed consents were recruited and blood samples were collected (EDTA plasma and serum), following overnight fasting. Final diagnosis of PDAC (either resection specimen, fine needle aspiration or biopsies of metastases) was confirmed by histopathology or ruled out in patients with non-pancreatic cancer disorder by a minimum clinical follow-up of 24 months. In case of controls, (IPMNs, non-IPMNs cystic lesions, chronic pancreatitis, acute pancreatitis and metastases of extra-pancreatic origins), final discharged diagnosis for 24 months follow-up considered as final diagnosis. A safety call concluded the study.

*Example 4: Primary outcomes*

The primary efficacy endpoint for METAPAC was set to be the diagnosis of PDAC with high specificity in a population at risk, employing two previously identified metabolite signatures (i- *and m-Metabolic signature).*

*Example 5: Laboratory procedures*

Whole-blood samples were processed to plasma and stored at -80°C before shipment to the central storage facility (University Medicine Greifswald). Serum CA19.9 levels were measured in a central laboratory (University Medicine Greifswald). Following collection of all biospecimens, 12 metabolites were analyzed for absolute concentration ($\mu$g/ml)

using LC-MS/MS according to Metabolon Method TAM217 at the Bioanalytical testing site of Metabolon Inc. Morrisville, NC, USA.

*Example 6: Detail targeted metabolite analysis*

The targeted metabolite panel measures 12 analytes: Histidine, Proline, Tryptophan, Ceramide (d18:1,C24:0), Ceramide (d18:2,C24:0), Lysophosphatidylethanolamine (C18:0), Lysophosphatidylethanolamine (C18:2), Phosphatidylethanolamine (C18:0), Sphingomyelin (d17:1,C16:0), Sphingomyelin (35:1), Sphingomyelin (41:2), and Sphingomyelin (d18:2,C17:0). Analyte concentrations are analyzed by LC-MS/MS (Metabolon method, TAM217).

Calibration samples are prepared at eight different concentration levels by spiking phosphate buffered saline with corresponding calibration spiking solutions. Calibration samples, study samples, and quality control samples are spiked with a solution of isotopically labeled internal standards and subjected to protein precipitation with an organic solution (2:1 methanol: dichloromethane). Following centrifugation, an aliquot of the organic supernatant were derivatized using dansyl chloride. The derivatized extract is diluted and injected onto an Agilent 1290 Infinity/SCIEX QTRAP 5500 LC-MS/MS system equipped with an Ascentis Express 90Å C18 column. The mass spectrometer is operated in positive mode using electrospray ionization (ESI). The peak areas of the respective product ions are measured against the peak area of the parent ions of the corresponding internal standard. Quantitation is performed using a weighted linear least squares regression analysis generated from fortified calibration standards prepared immediately prior to each run. Calculated concentrations are based on the analysis of 20.0 $\mu$L of plasma.

LC-MS/MS raw data was collected and processed using SCIEX software Analyst 1.7.3 and processed using SCIEX OS-MQ software v3.1.6. Data reduction was performed using Microsoft Excel for Microsoft 365 MSO.

Sample analysis was carried out in a 96-well plate format containing two calibration curves and six QC samples (2 replicates per QC level) per plate to monitor assay performance. Three levels of QC (Endogenous, Mx and ClinChek®) were prepared for TAM217. The Mx QC was prepared by reconstituting a lot of lyophilized plasma, with water and aliquoted into 1.5 ml vials. The Endogenous QC was prepared by aliquoting a pooled lot of human plasma procured from BioIVT. Endogenous QC was run at endogenous levels, no analyte spike was performed. The ClinChek® QCs were procured from Iris Technology ("ClinChek® Plasma Control for Amino Acids - Level I"). Accuracy was evaluated for the Endogenous and Mx QCs. Clinchek® QCs were evaluated using precision as no established concentrations were available. A note has been added for any samples with analytes that did not meet acceptance criteria. Analyte concentrations that fell below the limit of quantitation are extrapolated and given a comment of BLOQ. Analyte concentrations that fell above the limit of quantitation are extrapolated and given a comment of ALOQ. Analytes that cannot be extrapolated below the limit of quantitation and are considered not quantifiable and are recorded as N/Q as the concentration. The central storage facility, central laboratory, Metabolon Inc. as well as the statistician were blinded to clinical findings until metabolome and clinical database freezing.

*Example 7: Statistical analysis*

The study was designed to have ≥80% power to assess all the primary and secondary analyses to be achieved by enrollment of at least 250 PDAC patients.

The primary and secondary analyses were based on all available data without imputation for CA19.9 levels. To successfully transfer the technology, targeted metabolite analysis on a randomly selected plasma samples (N = 346) was performed using previously established LC-MS/MS methodology at Metanomics Health GmbH, Berlin, Germany (Mahajan et al. 2022) and quantification was correlated with LC-MS/MS analysis at Metabolon Inc. To reduce inter-platform and internal standard associated bias for absolute quantification of metabolites, covariate shift correction was performed utilizing linear regression on matched samples. A linear regression model was constructed for each metabolite, which allowed the determination of the correction slope and intercept. Bias was corrected for all samples, which were then subject to further analysis. The effectiveness correction was assessed using the Concordance Correlation Coefficient (CCC). All metabolite profiling data were log10-transformed to achieve an approximate normal distribution. The log10-transformed, auto-scaled and median imputed data was independently evaluated with previously constructed 12 metabolites plus CA19.9 signature (*i-Metabolite signature*) and 4 metabolites plus CA19.9 signature (*m-Metabolite signature*) (Mahajan et al. 2022). Prediction scores for each patient were evaluated for its potential to differentiate PDAC from controls (IPMNs, non-IPMNs cystic lesions, chronic pancreatitis, acute pancreatitis and metastases of extra-pancreatic origins). The optimal cutoffs for *i-* and *m-Metabolite signature* were retained from previous studies (Mahajan et al. 2022). Subsequently, the prediction scores of *i-* and *m-Metabolite signatures* were compared to CA19.9 alone. The diagnostic performance of *i-* and *m-Metabolite signature* were evaluated using 10-fold cross validation. To mimic a population cohort with a 1-20% incidence of PDAC, PDAC patients were randomly sampled 1000 times to achieve the target prevalence (bootstrapping analysis). Briefly, the dataset was stratified by disease status, and CA19.9 values in the PDAC group were divided into quartiles for balanced sampling. For each prevalence level, 1000 bootstrap iterations were conducted, with samples drawn from each quartile according to the required prevalence. When quartiles lacked sufficient samples, sampling with replacement was utilized. The resulting cohort was then evaluated for the diagnostic performance of *i-* and *m-Metabolite signatures.* To account for the enrichment design, inverse probability weighting (IPW) was used to refine the analysis without requiring model retraining. Sampling weights were determined by comparing the original prevalence of pancreatic ductal adenocarcinoma (PDAC) at roughly 1% to the enriched sample prevalence of 20%. This adjustment enables the calculation of model performance metrics that better represent expected outcomes in the target population, while still capitalizing on the statistical efficiency of the enriched design. To assess model performance, 1,000 bootstrap resampling iterations were conducted to estimate confidence intervals for key metrics, including the weighted area under the curve (AUC), ensuring reliable results.

The DeLong test was employed to compare the AUC of the two signatures. Performance metrics (AUC, sensitivity, specificity, positive predictive value (PPV), and negative predictive value (NPV) and balanced accuracy are presented as means and 95% CI. Planned subgroup analyses of the primary outcome were conducted for the performance of i- and *m-Metabolite signatures* in resectable PDAC (stage I-IIB), detectable CA19.9 (> 10 U/ml), CA19.9 (< 37 U/ml) and patients with < 10 U/ml CA19.9 status. A subgroup analysis was conducted to evaluate the differential diagnosis performance of the *i-* and *m-Metabolic signatures* in distinguishing PDAC from individual controls. Student's t-test was utilized to assess the differences in predictive scores, while the false discovery rate was adjusted using the Bonferroni correction for multiple comparisons. For the primary outcome, a two-sided P-value of <0.05 was considered to indicate statistical significance. All data processing, modeling and assessment of performances was performed using R (version 4.4.0).

Example 8 (Comparative Example): Comparison to Mahajan et al. (2022)

In Mahajn et al. (2022), analysis was performed using a logistic regression model with elastic net regularization (glmnet). The model was trained using 10-fold cross-validation with deviance as the performance measure.

Model Specifications

- Family: Binomial (Logistic Regression)
- Standardization: Yes
- Cross-validation: 10-fold
- Performance Measure: Deviance

Model Performance

- Degrees of Freedom (Df): 11
- Percent Deviance Explained: 64.3%
- Lambda (Regularization Parameter): 0.0329

Coefficient Analysis

The model identified the following significant features with their corresponding coefficients are shown in Table 5.

Linear Equation

For the standardized variables, the model of Mahajan et al (2022) can be expressed as:

$\log(p/(1-p)) = \beta1(CA19\_9) + \beta2(Proline) + \beta3(Tryptophan) + \beta4(Histidine) + \beta5(Lysophosphatidylethanolamine (C18:0)) + \beta6(Lysophosphatidylethanolamine (C18:2)) + \beta7(Sphingomyelin (d17:1,C16:0)) + \beta8(Sphingomyelin (d18:2,C17:0)) + \beta9(Sphingomyelin (35:1)) + B10(Sphingomyelin (41:2)) + \beta11(Phosphatidylethanolamine (d18:0,C22:6)) + \beta12(Ceramide (d18:2,C24:0)) + \beta13(Ceramide (d18: 1,C24:0))$, where p is the probability of the positive class.

The aforesaid model of Mahajan et al. 2022, however omitting pre-classification according to CA19.9 expression, was compared to the models of the present invention; resulting performance parameters are summarized in Table 6. Further, performance parametes for CA19.9 alone are included as well.

Clearly, the models of the present invention have a better performance; also, contrary to Mahajan et al., 2022, pre-classification based on CA19.9 expression is not required.

Literature

Ben-Ami, Roni, Qiao-Li Wang, Jinming Zhang, Julianna G Supplee, Johannes F Fahrmann, Roni Lehmann-Werman, Lauren K Brais, et al. 2023. "Protein Biomarkers and Alternatively Methylated Cell-Free DNA Detect Early Stage Pancreatic Cancer." Gut, December, gutjnl-2023-331074. doi.org/10.1136/gutjnl-2023-331074.

Boyd, Lenka N. C., Mahsoem Ali, Annalisa Comandatore, Ingrid Garajova, Laura Kam, Jisce R. Puik, Stephanie M. Fraga Rodrigues, et al. 2023. "Prediction Model for Early-Stage Pancreatic Cancer Using Routinely Measured Blood Biomarkers." JAMA Network Open 6 (8): e2331197. doi.org/10.1001/jamanetworkopen.2023.31197.

Brand, Randall E., Jan Persson, Svein Olav Bratlie, Daniel C. Chung, Bryson W. Katona, Alfredo Carrato, Marién Castillo, et al. 2022. "Detection of Early-Stage Pancreatic Ductal Adenocarcinoma From Blood Samples: Results of a Multiplex Biomarker Signature Validation Study." Clinical and Translational Gastroenterology 13 (3): e00468. doi.org/10.14309/ctg.0000000000000468.

Brand et al., Gut. 2007;56:1460-9

Byeon, Sooin, Matthew J. McKay, Mark P. Molloy, Anthony J. Gill, Jaswinder S. Samra, Anubhav Mittal, and Sumit Sahni. 2024. "Novel Serum Protein Biomarker Panel for Early Diagnosis of Pancreatic Cancer." International Journal of Cancer 155 (2): 365-71. doi.org/10.1002/ijc.34928.

Cao, Yingying, Rui Zhao, Kai Guo, Shuai Ren, Yaping Zhang, Zipeng Lu, Lei Tian, Tao Li, Xiao Chen, and Zhongqiu Wang. 2022. "Potential Metabolite Biomarkers for Early Detection of Stage-I Pancreatic Ductal Adenocarcinoma." Frontiers in Oncology 11 (January):744667. doi.org/10.3389/fonc.2021.744667.

Cohen, Jérémie F., Daniël A. Korevaar, Douglas G. Altman, David E. Bruns, Constantine A. Gatsonis, Lotty Hooft, Les Irwig, et al. 2016. "STARD 2015 Guidelines for Reporting Diagnostic Accuracy Studies: Explanation and Elaboration." BMJ Open 6 (11): e012799. doi.org/10.1136/bmjopen-2016-012799.

Collins, Gary S, Johannes B Reitsma, Douglas G Altman, and Karel Moons. 2015. "Transparent Reporting of a Multivariable Prediction Model for Individual Prognosis or Diagnosis (TRIPOD): The TRIPOD Statement." BMC Medicine 13 (1): 1. doi.org/10.1186/s12916-014-0241-z.

Del Chiaro et al., World J Gastroenterol 2014; 20:12118-12131

Digiacomo, Luca, Erica Quagliarini, Daniela Pozzi, Roberto Coppola, Giulio Caracciolo, and Damiano Caputo. 2023. "Stratifying Risk for Pancreatic Cancer by Multiplexed Blood Test." Cancers 15 (11): 2983. doi.org/10.3390/cancers15112983.

Fahrmann, Johannes F., C. Max Schmidt, Xiangying Mao, Ehsan Irajizad, Maureen Loftus, Jinming Zhang, Nikul Patel, et al. 2021. "Lead-Time Trajectory of CA19-9 as an Anchor Marker for Pancreatic Cancer Early Detection." Gastroenterology 160 (4): 1373-1383.e6. doi.org/10.1053/j.gastro.2020.11.052.

Firpo, Matthew A., Kenneth M. Boucher, Josh Bleicher, Gayatri D. Khanderao, Alessandra Rosati, Katherine E. Poruk, Sama Kamal, et al. 2023. "Multianalyte Serum Biomarker Panel for Early Detection of Pancreatic Adenocarcinoma." JCO Clinical Cancer Informatics, no. 7 (March), e2200160. doi.org/10.1200/CCI.22.00160.

Haab, Brian, Lu Qian, Ben Staal, Maneesh Jain, Johannes Fahrmann, Christine Worthington, Denise Prosser, et al. 2024. "A Rigorous Multi-Laboratory Study of Known PDAC Biomarkers Identifies Increased Sensitivity and Specificity over CA19-9 Alone." Cancer Letters 604 (November):217245. doi.org/10.1016/j.canlet.2024.217245.

Iwano, Tomohiko, Kentaro Yoshimura, Genki Watanabe, Ryo Saito, Sho Kiritani, Hiromichi Kawaida, Takeshi Moriguchi, et al. 2021. "High-Performance Collective Biomarker from Liquid Biopsy for Diagnosis of Pancreatic Cancer Based on Mass Spectrometry and Machine Learning." Journal of Cancer 12 (24): 7477-87. doi.org/10.7150/jca.63244.

Katona, Bryson W., Christine Worthington, Daniel Clay, Hannah Cincotta, Nuzhat A. Ahmad, Gregory G. Ginsberg, Michael L. Kochman, and Randall E. Brand. 2023. "Outcomes of the IMMray PanCan-d Test in High-Risk Individuals Undergoing Pancreatic Surveillance: Pragmatic Data and Lessons Learned." JCO Precision Oncology, no. 7 (September), e2300445. doi.org/10.1200/PO.23.00445.

Kim, Yoseop, Injoon Yeo, Iksoo Huh, Jaenyeon Kim, Dohyun Han, Jin-Young Jang, and Youngsoo Kim. 2021. "Development and Multiple Validation of the Protein Multi-Marker Panel for Diagnosis of Pancreatic Cancer." Clinical Cancer Research 27 (8): 2236-45. doi.org/10.1158/1078-0432.CCR-20-3929.

Kim et al., Sci Rep 2023;13(1):106

Llach et al., Cancer Manag Res 2020;12:743-58

Li, Tiandong, Junfen Xia, Huan Yun, Guiying Sun, Yajing Shen, Peng Wang, Jianxiang Shi, Keyan Wang, Hongwei Yang, and Hua Ye. 2023. "A Novel Autoantibody Signatures for Enhanced Clinical Diagnosis of Pancreatic Ductal Adenocarcinoma." Cancer Cell International 23 (1): 273. doi.org/10.1186/s12935-023-03107-1.

Mahajan, Ujjwal M., Bettina Oehrle, Simon Sirtl, Ahmed Alnatsha, Elisabetta Goni, Ivonne Regel, Georg Beyer, et al. 2022. "Independent Validation and Assay Standardization of Improved Metabolic Biomarker Signature to Differentiate Pancreatic Ductal Adenocarcinoma From Chronic Pancreatitis." Gastroenterology 163 (5): 1407-22. doi.org/10.1053/j.gastro.2022.07.047.

Mayerle, Julia, Holger Kalthoff, Regina Reszka, Beate Kamlage, Erik Peter, Bodo Schniewind, Sandra González Maldonado, et al. 2018. "Metabolic Biomarker Signature to Differentiate Pancreatic Ductal Adenocarcinoma from Chronic Pancreatitis." Gut 67 (1): 128-37. doi.org/10.1136/gutjnl-2016-312432.

Nakamura, Kota, Zhongxu Zhu, Souvick Roy, Eunsung Jun, Haiyong Han, Ruben M. Munoz, Satoshi Nishiwada, et al. 2022. "An Exosome-Based Transcriptomic Signature for Noninvasive, Early Detection of Patients With Pancreatic Ductal Adenocarcinoma: A Multicenter Cohort Study." Gastroenterology 163 (5): 1252-1266.e2. doi.org/10.1053/j.gastro.2022.06.090.

Pepe et al., J Natl Cancer Inst 2008;100(20):1432-8

Sahni, Sumit, Advait R. Pandya, William J. Hadden, Christopher B. Nahm, Sarah Maloney, Victoria Cook, James A. Toft, et al. 2021. "A Unique Urinary Metabolomic Signature for the Detection of Pancreatic Ductal Adenocarcinoma." International Journal of Cancer 148 (6): 1508-18. doi.org/10.1002/ijc.33368.

Seeger, Nico, Stefan Gutknecht, Irin Zschokke, Isabella Fleischmann, Nadja Roth, Jürg Metzger, Markus Weber, Stefan Breitenstein, and Lukasz Filip Grochola. 2024. "A Predictive Noninvasive Single-Nucleotide Variation-Based Biomarker Signature for Resectable Pancreatic Cancer: Protocol for a Prospective Validation Study." JMIR Research Protocols 13 (May):e54042. doi.org/10.2196/54042.

Shen, Xiaojing, Xiaolin Zhu, Hairong Liu, Rongtao Yuan, Qingyuan Guo, and Peng Zhao. 2024. "Leveraging Genomic Signatures of Oral Microbiome-Associated Antibiotic Resistance Genes for Diagnosing Pancreatic Cancer." Edited by Yash Gupta. PLOS ONE 19 (4): e0302361. doi.org/10.1371/journal.pone.0302361.

Singhi et al., Gastroenterology 2019;156(7):2024-40

Song, Jin, Lori J. Sokoll, Daniel W. Chan, and Zhen Zhang. 2021. "Validation of Serum Biomarkers That Complement CA19-9 in Detecting Early Pancreatic Cancer Using Electrochemiluminescent-Based Multiplex Immunoassays." Biomedicines 9 (12): 1897. doi.org/10.3390/biomedicines9121897.

Srivastava, Sudhir, and Paul D. Wagner. 2020. "The Early Detection Research Network: A National Infrastructure to Support the Discovery, Development, and Validation of Cancer Biomarkers." Cancer Epidemiology, Biomarkers & Prevention: A Publication of the American Association for Cancer Research, Cosponsored by the American Society of Preventive Oncology 29 (12): 2401-10. doi.org/10.1158/1055-9965.EPI-20-0237.

Wen, Yan-Rong, Xia-Wen Lin, Yu-Wen Zhou, Lei Xu, Jun-Li Zhang, Cui-Ying Chen, and Jian He. 2024. "N-Glycan Biosignatures as a Potential Diagnostic Biomarker for Early-Stage Pancreatic Cancer." World Journal of Gastrointestinal Oncology 16 (3): 659-69. doi.org/10.4251/wjgo.v16.i3.659.

Wu, Huanwen, Shiwei Guo, Xiaoding Liu, Yatong Li, Zhixi Su, Qiye He, Xiaoqian Liu, et al. 2022. "Noninvasive Detection of Pancreatic Ductal Adenocarcinoma Using the Methylation Signature of Circulating Tumour DNA." BMC Medicine 20 (1): 458. doi.org/10.1186/s 12916-022-02647-z.

Xu, Caiming, Eunsung Jun, Yoshinaga Okugawa, Yuji Toiyama, Erkut Borazanci, John Bolton, Akinobu Taketomi, et al. 2024. "A Circulating Panel of circRNA Biomarkers for the Noninvasive and Early Detection of Pancreatic Ductal Adenocarcinoma." Gastroenterology 166 (1): 178-190.e16. doi.org/10.1053/j.gastro.2023.09.050.

Yu, Jingru, Alexander Ploner, Maximilian Kordes, Matthias Löhr, Magnus Nilsson, Maria Evangelina Lopez De Maturana, Lidia Estudillo, et al. 2021. "Plasma Protein Biomarkers for Early Detection of Pancreatic Ductal Adenocarcinoma." International Journal of Cancer 148 (8): 2048-58. doi.org/10.1002/ijc.33464.

Zweig 1993, Clin. Chem. 39:561-577

Table 2: Preferred coefficient values for formula (I) when using the m-metabolic signature

| Biomarker | variable name | coefficient name | coefficient value | | | |
|---|---|---|---|---|---|---|
| | | | | 1 significant digit | 2 significant digits | 3 significant digits |
| | | $\beta_0$ | 0.271543 | 0.3 | 0.27 | 0.272 |
| CA19.9 | $X_1$ | $\beta_1$ | 1.988441 | 2 | 2.0 | 1.99 |
| Lysophosphatidylethanolamine (C18:0) | $X_2$ | $\beta_2$ | -1.011733 | -1 | -1.0 | -1.01 |
| Sphingomyelin (d17:1,C16:0) | $X_3$ | $\beta_3$ | 1.224207 | 1 | 1.2 | 1.22 |

(continued)

| Biomarker | variable name | coefficient name | coefficient value | | | |
|---|---|---|---|---|---|---|
| | | | | 1 significant digit | 2 significant digits | 3 significant digits |
| Phosphatidylethanolamine (d18:0,C22:6) | $X_4$ | $\beta_4$ | 0.911924 | 1 | 0.9 | 0.91 |
| Ceramide (d18:2,C24:0) | $X_5$ | $\beta_5$ | -0.559065 | 0.6 | 0.56 | 0.559 |

Table 3: Preferred coefficient values for formula (I) when using the i-metabolic signature

| Biomarker | variable name | coefficient name | coefficient value | | | |
|---|---|---|---|---|---|---|
| | | | | 1 significant digit | 2 significant digits | 3 significant digits |
| | | $\beta_0$ | 0.266311 | 0.3 | 0.27 | 0.266 |
| CA19.9 | $X_1$ | $\beta_1$ | 1.968635 | 2 | 2.0 | 1.97 |
| Proline | $X_2$ | $\beta_2$ | -0.598584 | -0.6 | -0.60 | -0.599 |
| Tryptophan | $X_3$ | $\beta_3$ | -0.410069 | -0.4 | -0.41 | -0.410 |
| Histidine | $X_4$ | $\beta_4$ | -0.367436 | -0.4 | -0.37 | -0.367 |
| Lysophosphatidylethanolamine (C18:0) | $X_5$ | $\beta_5$ | -0.817823 | -0.8 | -0.82 | -0.818 |
| Lysophosphatidylethanolamine (C 18:2) | $X_6$ | $\beta_6$ | 0.063470 | 0.06 | 0.063 | 0.0634 |
| Sphingomyelin (d17:1,C16:0) | $X_7$ | $\beta_7$ | 0.678383 | 0.7 | 0.68 | 0.678 |
| Sphingomyelin (d18:2,C17:0) | $X_8$ | $\beta_8$ | 0.170432 | 0.2 | 0.17 | 0.170 |
| Sphingomyelin (35:1) | $X_9$ | $\beta_9$ | 0.434002 | 0.4 | 0.43 | 0.434 |
| Sphingomyelin (41:2) | $X_{10}$ | $\beta_{10}$ | -0.056717 | -0.06 | -0.057 | -0.0567 |
| Phosphatidylethanolamine (d18:0,C22:6) | $X_{11}$ | $\beta_{11}$ | 0.801986 | 0.8 | 0.80 | 0.802 |
| Ceramide (d18:2,C24:0) | $X_{12}$ | $\beta_{12}$ | -0.727832 | -0.7 | -0.73 | -0.728 |
| Ceramide (d18:1,C24:0) | $X_{13}$ | $\beta_{13}$ | 0.417335 | 0.4 | 0.42 | 0.417 |

Table 4a: Training data for training the m-metabolic signature; NO: sample number; PDAC: pancreatic ductal adenocarcinoma; CP: chronic pancreatitis; LPE: Lysophosphatidylethanolamine; SM: Sphingomyelin; PE: Phosphatidylethanolamine; Cer: Ceramide, re.: resectable.

| No | PDAC | Diagnosis | Gender | Tumor Grade | res. | Disease status | CA19.9 | LPE (C18:0) | SM (d17:1,C 16:0) | PE (d18:0,C 22:6) | Cer (d18:2,C 24:0) |
|---|---|---|---|---|---|---|---|---|---|---|---|
| 1 | no | Chronic pancreatitis | male | | | CP | -0.7153 | 0.3250 | -1.2919 | -1.0680 | -0.3574 |
| 2 | yes | Pancreatic cancer | female | IIA | yes | PDAC | 0.8265 | -0.6156 | 1.9789 | 1.1570 | -1.4609 |
| 3 | yes | Pancreatic cancer | female | IIB | yes | PDAC | 0.3632 | 2.2223 | 3.2476 | 1.2852 | 1.3276 |

(continued)

| No | PDAC | Diagnosis | Gender | Tumor Grade | res. | Disease status | CA19.9 | LPE (C18:0) | SM (d17:1,C 16:0) | PE (d18:0,C 22:6) | Cer (d18:2,C 24:0) |
|---|---|---|---|---|---|---|---|---|---|---|---|
| 4 | no | Chronic pancreatitis | male | | | CP | -0.4033 | 1.6250 | -0.6233 | 0.5091 | 1.8159 |
| 5 | yes | Pancreatic cancer | male | IIB | yes | PDAC | 1.5796 | 0.7901 | 1.2186 | 1.4648 | 1.3194 |
| 6 | yes | Pancreatic cancer | male | III | no | PDAC | -0.5011 | -1.7586 | -0.3318 | -0.7425 | -1.3058 |
| 7 | yes | Pancreatic cancer | male | IIB | yes | PDAC | 1.0928 | -0.0088 | -0.5128 | 1.1063 | -1.1101 |
| 8 | no | Chronic pancreatitis | male | | | CP | -0.7820 | -0.5628 | -0.8563 | -0.1342 | -0.6375 |
| 9 | no | Chronic pancreatitis | male | | | CP | -0.4538 | 0.7114 | -0.7779 | 0.2378 | -1.6348 |
| 10 | yes | Pancreatic cancer | male | III | no | PDAC | 0.8278 | 0.3436 | 0.1543 | -1.3817 | 0.2161 |
| 11 | no | Chronic pancreatitis | male | | | CP | 0.2293 | -1.4429 | 0.0442 | -1.2541 | -1.6668 |
| 12 | yes | Pancreatic cancer | female | III | no | PDAC | 1.4153 | -0.3953 | 2.7669 | 1.0408 | -0.6548 |
| 13 | no | Chronic pancreatitis | male | | | CP | -0.2658 | 0.4713 | 0.3261 | -0.9003 | 0.8354 |
| 14 | yes | Pancreatic cancer | female | IV | no | PDAC | 0.9643 | -0.6503 | -0.4884 | 0.1631 | 0.0669 |
| 15 | yes | Pancreatic cancer | female | IIB | yes | PDAC | 0.0652 | 0.8234 | 1.0505 | 1.0317 | -0.3843 |
| 16 | yes | Pancreatic cancer | male | IIB | yes | PDAC | 1.8130 | -1.4402 | 0.5954 | 1.4535 | -0.7217 |
| 17 | no | Chronic pancreatitis | male | | | CP | -0.6626 | 0.3866 | -0.7078 | -1.5807 | 1.0185 |
| 18 | yes | Pancreatic cancer | male | III | no | PDAC | -0.9046 | -0.3059 | 0.9418 | 0.2502 | 0.2070 |
| 19 | no | Chronic pancreatitis | male | | | CP | -1.0862 | -0.4945 | -0.8948 | -0.3114 | 0.1554 |
| 20 | yes | Pancreatic cancer | male | IIB | yes | PDAC | 1.6033 | -0.7460 | 0.1569 | -0.0994 | -0.0760 |
| 21 | yes | Pancreatic cancer | female | IIB | yes | PDAC | 1.2595 | -1.2274 | -0.2973 | 0.3328 | -1.2639 |
| 22 | yes | Pancreatic cancer | male | IIB | yes | PDAC | 2.0076 | -0.6647 | 1.1998 | 1.0067 | 0.0209 |
| 23 | yes | Pancreatic cancer | male | IIB | yes | PDAC | 0.9774 | 0.9780 | 0.2711 | 0.7198 | 1.3082 |
| 24 | yes | Pancreatic cancer | female | IIA | yes | PDAC | 0.2107 | -0.8341 | 1.4073 | 0.7192 | -0.9037 |
| 25 | no | Chronic pancreatitis | male | | | CP | -0.9541 | 0.3271 | 1.3715 | -0.9366 | 0.7028 |

(continued)

| No | PDAC | Diagnosis | Gender | Tumor Grade | res. | Disease status | CA19.9 | LPE (C18:0) | SM (d17:1,C 16:0) | PE (d18:0,C 22:6) | Cer (d18:2,C 24:0) |
|---|---|---|---|---|---|---|---|---|---|---|---|
| 26 | no | Chronic pancreatitis | male | | | CP | -0.8954 | -1.2961 | -0.3318 | 0.6209 | -1.5785 |
| 27 | no | Chronic pancreatitis | male | | | CP | -0.8686 | 0.5644 | -1.0510 | -1.9980 | -0.7169 |
| 28 | no | Chronic pancreatitis | male | | | CP | -1.3907 | 0.1382 | -1.3693 | 0.6325 | 1.0774 |
| 29 | yes | Pancreatic cancer | female | IIB | yes | PDAC | 0.2040 | 0.1335 | 0.8828 | 0.6965 | 0.6829 |
| 30 | no | Chronic pancreatitis | male | | | CP | -0.3729 | -0.5086 | 0.4227 | -0.7973 | 0.4212 |
| 31 | no | Chronic pancreatitis | female | | | CP | -0.6682 | 1.8159 | 1.0136 | 0.6627 | 1.7042 |
| 32 | no | Chronic pancreatitis | male | | | CP | -0.4359 | 0.1463 | -0.6793 | 0.1801 | 1.2062 |
| 33 | yes | Pancreatic cancer | female | IIB | yes | PDAC | 0.4719 | 0.6871 | 1.3449 | 0.2347 | 1.1366 |
| 34 | no | Chronic pancreatitis | male | | | CP | -0.1848 | 0.0139 | -0.4931 | 1.1455 | 1.2794 |
| 35 | yes | Pancreatic cancer | female | IIA | yes | PDAC | -0.1419 | -2.1925 | 0.8330 | 1.7239 | -1.4249 |
| 36 | yes | Pancreatic cancer | male | IIA | yes | PDAC | 0.5643 | -1.2632 | -0.4730 | -0.6381 | -2.3440 |
| 37 | no | Chronic pancreatitis | male | | | CP | -0.9180 | -0.9583 | -1.0790 | -0.3905 | -1.0310 |
| 38 | no | Chronic pancreatitis | male | | | CP | -1.3397 | 0.8802 | -0.3054 | 1.0554 | 0.0609 |
| 39 | no | Chronic pancreatitis | female | | | CP | -0.8433 | 1.7047 | 0.7882 | 1.1807 | 0.8782 |
| 40 | yes | Pancreatic cancer | female | III | no | PDAC | 1.8909 | 2.0295 | 0.7435 | 1.6845 | 0.7892 |
| 41 | no | Chronic pancreatitis | male | | | CP | -0.2355 | 1.0220 | -0.9705 | 0.6859 | 0.9237 |
| 42 | no | Chronic pancreatitis | male | | | CP | -0.9437 | -0.0325 | 0.0529 | -1.6708 | 0.4138 |
| 43 | yes | Pancreatic cancer | female | IIB | yes | PDAC | 1.3877 | -1.5435 | 0.3007 | -0.6461 | -1.8257 |
| 44 | yes | Pancreatic cancer | female | IIB | yes | PDAC | 1.7122 | -1.3792 | -0.9227 | -0.7553 | -1.2540 |
| 45 | yes | Pancreatic cancer | male | IIA | yes | PDAC | 1.9671 | 0.9110 | 2.0450 | -0.3780 | 0.1956 |
| 46 | yes | Pancreatic cancer | male | III | no | PDAC | 1.0383 | -1.4809 | -0.5332 | -0.8374 | 0.0777 |
| 47 | yes | Pancreatic cancer | male | IIA | yes | PDAC | 0.6963 | -0.8902 | -0.7855 | 0.3010 | 0.4159 |

(continued)

| No | PDAC | Diagnosis | Gender | Tumor Grade | res. | Disease status | CA19.9 | LPE (C18:0) | SM (d17:1,C 16:0) | PE (d18:0,C 22:6) | Cer (d18:2,C 24:0) |
|---|---|---|---|---|---|---|---|---|---|---|---|
| 48 | no | Chronic pancreatitis | male | | | CP | -0.3416 | -0.1796 | 0.0267 | -1.4744 | 1.0970 |
| 49 | no | Chronic pancreatitis | male | | | CP | -0.5129 | 1.0938 | -0.3356 | -0.6555 | 2.0733 |
| 50 | yes | Pancreatic cancer | male | IIB | yes | PDAC | -0.0473 | -0.2851 | -0.2019 | 0.0221 | 0.7009 |
| 51 | no | Chronic pancreatitis | male | | | CP | -0.7286 | 1.0035 | -1.5554 | -0.7900 | 0.1215 |
| 52 | no | Chronic pancreatitis | male | | | CP | -0.8194 | -1.3052 | 0.0921 | -3.1066 | 0.5150 |
| 53 | no | Chronic pancreatitis | male | | | CP | -1.1455 | -0.4872 | -0.8547 | -0.4750 | -0.1271 |
| 54 | yes | Pancreatic cancer | male | IIB | yes | PDAC | 0.4518 | -0.2383 | -1.7813 | -0.7298 | -1.1064 |
| 55 | no | Chronic pancreatitis | male | | | CP | -0.2946 | 0.4073 | 0.5692 | -0.4762 | -0.0159 |
| 56 | yes | Pancreatic cancer | female | III | no | PDAC | 2.5721 | -0.9009 | -0.6998 | 1.0624 | -2.1861 |
| 57 | no | Chronic pancreatitis | male | | | CP | -1.3397 | -0.1401 | -0.2589 | -1.7934 | -0.7120 |
| 58 | no | Chronic pancreatitis | male | | | CP | -0.8863 | 0.2251 | -0.9918 | 0.2386 | 1.2741 |
| 59 | yes | Pancreatic cancer | male | III | no | PDAC | -0.1828 | -0.3314 | 0.3950 | -1.1890 | -0.0098 |
| 60 | no | Chronic pancreatitis | male | | | CP | -0.2776 | -0.2987 | 0.9186 | -0.5589 | 0.6426 |
| 61 | no | Chronic pancreatitis | male | | | CP | -0.4427 | 0.3975 | -1.2285 | 0.1639 | -0.3772 |
| 62 | yes | Pancreatic cancer | male | IIB | yes | PDAC | 1.2616 | -0.2901 | -0.3566 | 1.4754 | 0.6407 |
| 63 | no | Chronic pancreatitis | male | | | CP | -0.5817 | 0.1013 | 0.6219 | 0.6209 | -1.5060 |
| 64 | no | Chronic pancreatitis | male | | | CP | -0.7216 | 0.2158 | -0.1187 | -2.7588 | 0.1991 |
| 65 | no | Chronic pancreatitis | male | | | CP | -1.5356 | 0.9572 | -0.1923 | -0.0047 | 0.2954 |
| 66 | yes | Pancreatic cancer | female | IIA | yes | PDAC | -1.4331 | -1.1822 | 0.6429 | 1.1109 | -1.3463 |
| 67 | yes | Pancreatic cancer | male | IIB | yes | PDAC | 0.3638 | -0.1835 | 0.8147 | 1.2207 | -0.0709 |
| 68 | no | Chronic pancreatitis | male | | | CP | 0.2371 | -0.3109 | -0.1762 | -2.2180 | -0.6753 |
| 69 | yes | Pancreatic cancer | male | IIB | yes | PDAC | 0.1125 | 1.8367 | 1.3776 | 2.2746 | 1.7890 |

(continued)

| No | PDAC | Diagnosis | Gender | Tumor Grade | res. | Disease status | CA19.9 | LPE (C18:0) | SM (d17:1,C 16:0) | PE (d18:0,C 22:6) | Cer (d18:2,C 24:0) |
|---|---|---|---|---|---|---|---|---|---|---|---|
| 70 | no | Chronic pancreatitis | female | | | CP | -0.8344 | 1.5429 | -1.9913 | 0.7882 | -1.0915 |
| 71 | no | Chronic pancreatitis | male | | | CP | -2.1872 | -0.1905 | -1.2956 | -0.0757 | 0.6223 |
| 72 | yes | Pancreatic cancer | female | IIB | yes | PDAC | -0.0711 | -1.0000 | -0.3617 | -0.4055 | -0.7185 |
| 73 | no | Chronic pancreatitis | female | | | CP | -0.3584 | 1.4796 | -0.2382 | 0.5962 | 1.0477 |
| 74 | yes | Pancreatic cancer | male | III | no | PDAC | 0.7788 | 0.8779 | 0.4166 | -0.4055 | -0.0283 |
| 75 | no | Chronic pancreatitis | male | | | CP | -0.8774 | -0.2163 | -0.7404 | -0.5066 | 0.5612 |
| 76 | no | Chronic pancreatitis | male | | | CP | -1.4482 | -1.2722 | -1.8212 | -0.6826 | -1.0310 |
| 77 | yes | Pancreatic cancer | male | IIB | yes | PDAC | 0.2502 | 0.1677 | 0.6150 | -0.4428 | 0.5463 |
| 78 | no | Chronic pancreatitis | male | | | CP | -0.4129 | 0.5107 | 0.0680 | -0.8003 | 1.5337 |
| 79 | yes | Pancreatic cancer | female | IIB | yes | PDAC | 0.2348 | 0.6377 | 3.5352 | 1.0750 | 1.5728 |
| 80 | no | Chronic pancreatitis | female | | | CP | -0.9238 | -0.0539 | -0.4430 | -1.6056 | 1.5115 |
| 81 | no | Chronic pancreatitis | male | | | CP | -1.0725 | 0.0155 | -1.4962 | -0.1351 | -0.4534 |
| 82 | no | Chronic pancreatitis | female | | | CP | -0.7153 | 1.2320 | -0.3598 | -0.6978 | 1.2365 |
| 83 | yes | Pancreatic cancer | female | IIB | yes | PDAC | 0.7723 | 0.4603 | 0.9925 | 0.2883 | -0.3814 |
| 84 | yes | Pancreatic cancer | female | IIA | yes | PDAC | 0.0797 | 0.0062 | 0.5185 | 0.0096 | -0.3017 |
| 85 | yes | Pancreatic cancer | male | IIB | yes | PDAC | 0.4419 | -0.6151 | -0.2979 | -0.6065 | -0.0900 |
| 86 | no | Chronic pancreatitis | male | | | CP | -0.5680 | 1.6937 | 0.8843 | 0.2943 | 1.9978 |
| 87 | yes | Pancreatic cancer | female | IIB | yes | PDAC | 0.9562 | -0.1870 | 0.3860 | 0.1873 | -1.0166 |
| 88 | yes | Pancreatic cancer | male | IIA | yes | PDAC | 1.7724 | 0.1795 | -0.8384 | 1.3232 | 0.5332 |
| 89 | no | Chronic pancreatitis | male | | | CP | -1.0214 | 0.7843 | -1.1907 | -0.6555 | 0.9037 |
| 90 | no | Chronic pancreatitis | male | | | CP | -0.3788 | -0.7851 | -1.5992 | -0.2505 | 0.1449 |
| 91 | yes | Pancreatic cancer | female | IB | yes | PDAC | -0.4566 | -0.6115 | 0.7365 | 0.5805 | -0.6992 |

(continued)

| No | PDAC | Diagnosis | Gender | Tumor Grade | res. | Disease status | CA19.9 | LPE (C18:0) | SM (d17:1,C 16:0) | PE (d18:0,C 22:6) | Cer (d18:2,C 24:0) |
|---|---|---|---|---|---|---|---|---|---|---|---|
| 92 | yes | Pancreatic cancer | male | IIA | yes | PDAC | 0.1843 | -1.9662 | -0.6240 | 1.7239 | -2.2324 |
| 93 | no | Chronic pancreatitis | male | | | CP | -0.6065 | -0.8237 | 0.1501 | 1.1190 | -0.8321 |
| 94 | yes | Pancreatic cancer | female | IIB | yes | PDAC | 2.0393 | -0.8035 | 1.0264 | 0.0088 | -0.3406 |
| 95 | no | Chronic pancreatitis | female | | | CP | -1.7368 | 0.4521 | -0.0750 | 0.1768 | 0.2421 |
| 96 | no | Chronic pancreatitis | male | | | CP | -1.0336 | 0.0856 | -0.2037 | -1.1016 | 1.1502 |
| 97 | no | Chronic pancreatitis | male | | | CP | -1.1003 | 3.5444 | -0.1644 | 0.5170 | 1.7543 |
| 98 | no | Chronic pancreatitis | female | | | CP | -1.3470 | 0.0639 | 0.4446 | -0.3555 | 0.4128 |
| 99 | no | Chronic pancreatitis | male | | | CP | -0.2996 | -0.3145 | -0.8641 | -0.3421 | 0.9107 |
| 100 | no | Chronic pancreatitis | male | | | CP | -0.4259 | 1.1928 | 0.1631 | 1.0926 | -0.5575 |
| 101 | yes | Pancreatic cancer | male | IV | no | PDAC | 0.0407 | -0.3420 | -0.5339 | -0.3223 | -0.8170 |
| 102 | yes | Pancreatic cancer | male | IIB | yes | PDAC | 1.3599 | -1.3846 | 1.5375 | 1.5181 | -0.7509 |
| 103 | no | Chronic pancreatitis | male | | | CP | -1.4766 | -0.9150 | -0.6677 | -0.0495 | 0.5352 |
| 104 | yes | Pancreatic cancer | male | IIB | yes | PDAC | 0.7488 | 0.3033 | 0.2113 | 1.3232 | -1.1649 |
| 105 | yes | Pancreatic cancer | male | IV | no | PDAC | -0.3334 | -0.1840 | -0.3496 | -0.6964 | -0.4665 |
| 106 | no | Chronic pancreatitis | male | | | CP | 0.9365 | -0.3567 | -0.4643 | -0.9113 | 0.0955 |
| 107 | no | Chronic pancreatitis | female | | | CP | -0.2704 | -0.3200 | -0.3098 | -0.3792 | 0.1062 |
| 108 | yes | Pancreatic cancer | male | IIA | yes | PDAC | 1.7663 | 0.8876 | -0.0175 | -0.3278 | 0.6184 |
| 109 | no | Chronic pancreatitis | male | | | CP | -0.4820 | 0.3293 | -1.1553 | -2.0911 | 0.4483 |
| 110 | no | Chronic pancreatitis | male | | | CP | -0.8541 | 1.2475 | -0.7984 | 0.4358 | 0.7487 |
| 111 | yes | Pancreatic cancer | male | III | no | PDAC | 0.5476 | -0.9768 | 1.0901 | 0.1760 | 0.2778 |
| 112 | no | Chronic pancreatitis | male | | | CP | 0.9417 | 2.4678 | 0.8440 | -1.2081 | 0.5919 |
| 113 | yes | Pancreatic cancer | male | IV | no | PDAC | 0.2197 | 0.5759 | -1.5699 | 0.3474 | -0.0558 |

(continued)

| No | PDAC | Diagnosis | Gender | Tumor Grade | res. | Disease status | CA19.9 | LPE (C18:0) | SM (d17:1,C 16:0) | PE (d18:0,C 22:6) | Cer (d18:2,C 24:0) |
|---|---|---|---|---|---|---|---|---|---|---|---|
| 114 | yes | Pancreatic cancer | female | III | no | PDAC | -2.1872 | -1.3393 | 0.5933 | 0.7134 | -1.1083 |
| 115 | no | Chronic pancreatitis | male | | | CP | -0.7197 | 0.0993 | -1.0384 | -0.8255 | -0.5243 |
| 116 | yes | Pancreatic cancer | male | IIA | yes | PDAC | 0.3963 | 0.0155 | 0.8342 | 0.7501 | -1.1821 |
| 117 | no | Chronic pancreatitis | male | | | CP | -0.2373 | 0.3282 | 0.5793 | -1.1416 | 0.7754 |
| 118 | yes | Pancreatic cancer | male | IIB | yes | PDAC | 1.2605 | -1.8740 | 0.5350 | 1.5710 | -1.1725 |
| 119 | no | Chronic pancreatitis | male | | | CP | -0.3818 | -1.2123 | -1.2403 | -1.4966 | -0.7640 |
| 120 | no | Chronic pancreatitis | male | | | CP | 0.2261 | 2.6423 | 1.0371 | 1.3839 | 2.3691 |
| 121 | no | Chronic pancreatitis | male | | | CP | -0.2386 | -0.8106 | -0.4128 | 0.9047 | -0.7330 |
| 122 | no | Chronic pancreatitis | male | | | CP | -0.4858 | 0.0336 | -0.1169 | -1.3692 | 0.5840 |
| 123 | yes | Pancreatic cancer | female | IIB | yes | PDAC | -1.1149 | -0.8523 | 0.3095 | 0.1467 | -0.2648 |
| 124 | yes | Pancreatic cancer | female | IIA | yes | PDAC | -0.1143 | 0.5255 | 2.1615 | 1.4754 | -0.2729 |
| 125 | yes | Pancreatic cancer | male | III | no | PDAC | 0.3708 | -0.1036 | 1.3455 | 0.7910 | -1.8775 |
| 126 | yes | Pancreatic cancer | male | IIB | yes | PDAC | 0.7584 | -0.3332 | 1.7641 | 1.8264 | -0.1713 |
| 127 | yes | Pancreatic cancer | female | IIB | yes | PDAC | -0.0430 | -0.8612 | 0.9970 | 2.0335 | 0.0896 |
| 128 | yes | Pancreatic cancer | female | IIB | yes | PDAC | -1.2723 | 0.6719 | 1.0345 | 0.9955 | -0.4665 |
| 129 | yes | Pancreatic cancer | female | IB | yes | PDAC | 1.4139 | -0.8579 | 1.0708 | 0.5384 | 0.4546 |
| 130 | no | Chronic pancreatitis | male | | | CP | -0.5680 | -0.0676 | 0.4850 | -1.3254 | 0.2432 |
| 131 | yes | Pancreatic cancer | female | IIB | yes | PDAC | 0.7579 | -0.1061 | 0.5090 | 0.3947 | 0.7983 |
| 132 | no | Chronic pancreatitis | male | | | CP | -0.4820 | 0.3444 | -0.8485 | -1.3489 | 1.2496 |
| 133 | no | Chronic pancreatitis | male | | | CP | -0.7031 | 1.5590 | 0.2250 | -0.2695 | 1.6320 |
| 134 | no | Chronic pancreatitis | male | | | CP | 0.3499 | 0.7931 | -1.5022 | -0.3125 | 0.4327 |
| 135 | no | Chronic pancreatitis | male | | | CP | -0.6615 | 0.3109 | -1.0117 | -1.0713 | 0.2184 |

(continued)

| No | PDAC | Diagnosis | Gender | Tumor Grade | res. | Disease status | CA19.9 | LPE (C18:0) | SM (d17:1,C 16:0) | PE (d18:0,C 22:6) | Cer (d18:2,C 24:0) |
|---|---|---|---|---|---|---|---|---|---|---|---|
| 136 | no | Chronic pancreatitis | female | | | CP | -0.9141 | 0.9188 | -0.8532 | 0.1541 | 1.0268 |
| 137 | no | Chronic pancreatitis | female | | | CP | 0.4344 | -0.3609 | -1.8097 | -0.2029 | 0.0075 |
| 138 | yes | Pancreatic cancer | male | IIB | yes | PDAC | 2.0184 | 0.0575 | -0.8579 | 0.5999 | 0.7800 |
| 139 | no | Chronic pancreatitis | male | | | CP | -0.8516 | 0.8423 | 0.7228 | -0.1807 | 1.4063 |
| 140 | yes | Pancreatic cancer | female | III | no | PDAC | 0.4099 | 1.2603 | 1.0425 | -0.5004 | 1.3706 |
| 141 | yes | Pancreatic cancer | male | IIA | yes | PDAC | -0.3307 | -0.6653 | -0.9251 | -0.1578 | -0.6220 |
| 142 | yes | Pancreatic cancer | female | III | no | PDAC | -0.3444 | 0.0066 | 0.5413 | 0.5352 | -0.5079 |
| 143 | no | Chronic pancreatitis | male | | | CP | -0.8863 | 0.0147 | -1.6696 | -1.1343 | -1.3936 |
| 144 | yes | Pancreatic cancer | male | IIB | yes | PDAC | 1.1464 | -0.2370 | -1.7154 | -0.2726 | -1.5541 |
| 145 | no | Chronic pancreatitis | male | | | CP | -1.0214 | -0.3813 | -0.7901 | -0.5987 | -0.1452 |
| 146 | no | Chronic pancreatitis | male | | | CP | -0.8516 | -1.2728 | -1.1474 | -1.0809 | -1.5564 |
| 147 | yes | Pancreatic cancer | female | IIA | yes | PDAC | 1.8525 | -0.3447 | 0.4134 | -0.4570 | -0.5168 |
| 148 | yes | Pancreatic cancer | male | III | no | PDAC | 1.1276 | -0.1175 | 0.7886 | 0.5943 | 0.3969 |
| 149 | yes | Pancreatic cancer | male | IIA | yes | PDAC | 0.5318 | -2.3586 | -0.7300 | 1.1680 | -1.3058 |
| 150 | yes | Pancreatic cancer | male | III | no | PDAC | 0.4344 | -0.5593 | -0.4776 | -0.9730 | -0.6864 |
| 151 | yes | Pancreatic cancer | female | III | no | PDAC | -0.3254 | 0.2931 | 0.4213 | -0.2234 | 0.0979 |
| 152 | yes | Pancreatic cancer | female | IIB | yes | PDAC | 0.8209 | -0.7471 | 0.2149 | 0.0529 | -1.2639 |
| 153 | yes | Pancreatic cancer | male | III | no | PDAC | 2.6638 | -0.0556 | 0.6903 | -0.4871 | -0.4200 |
| 154 | no | Chronic pancreatitis | female | | | CP | -0.7735 | 2.4362 | -0.2284 | 1.4513 | 0.0404 |
| 155 | no | Chronic pancreatitis | male | | | CP | 0.4643 | 0.1807 | -0.2793 | 0.0061 | -0.0321 |
| 156 | yes | Pancreatic cancer | male | IV | no | PDAC | 2.3825 | -1.6985 | 0.1017 | 0.0958 | -1.6257 |

Table 4b: Additional training data for training the i-metabolic signature; NO: sample number; Pro: proline; Trp: tryptophan; His: histidine; LPE: Lysophosphatidylethanolamine; SM: Sphingomyelin; PE: Phosphatidylethanolamine; Cer: Ceramide.

| No | Pro | Trp | His | LPE (C 18:2) | SM (d18:2, C17:0) | SM (35:1) | SM (41:2) | Cer (d18:1,C24:0) |
|---|---|---|---|---|---|---|---|---|
| 1 | -0.5936 | -2.8832 | -0.2163 | 0.3607 | -0.9663 | -1.2007 | -1.1621 | -0.6451 |
| 2 | -0.1274 | 1.2274 | 0.9438 | -0.7958 | 1.8069 | 1.7643 | 1.7579 | -1.7332 |
| 3 | -1.0157 | -0.6461 | 0.5665 | -0.0373 | 3.4621 | 2.9903 | 2.5141 | 0.2577 |
| 4 | 1.0369 | 1.6854 | 1.9526 | 0.7300 | -0.5395 | -0.6995 | 0.2213 | 1.8292 |
| 5 | -0.9921 | 0.5238 | -0.3174 | 0.2179 | 1.1128 | 1.0222 | 1.2935 | 1.6147 |
| 6 | -0.0128 | -0.6989 | -0.6339 | -0.7066 | -0.1133 | 0.3165 | -0.2479 | -1.6793 |
| 7 | -0.1447 | 0.9647 | 0.9895 | -1.7368 | 0.2066 | -0.2132 | -0.2816 | -1.3002 |
| 8 | 0.5110 | -0.4576 | -1.1358 | -0.5360 | -1.2835 | -0.3680 | -1.0823 | -1.1026 |
| 9 | 2.6973 | -0.2972 | 0.3386 | 0.8025 | -0.6830 | -0.7492 | -0.6320 | -1.7738 |
| 10 | 1.3033 | 0.7392 | -0.3085 | 1.0775 | -0.1895 | 0.1373 | 0.1196 | 0.9286 |
| 11 | 1.3433 | 1.4158 | 0.2230 | 0.8100 | -0.2161 | -0.1879 | -0.2556 | -1.9865 |
| 12 | 1.5158 | -1.0230 | -0.1138 | -1.2036 | 2.7165 | 2.6421 | 1.8052 | -0.3921 |
| 13 | -0.1312 | 1.4685 | 0.8276 | 1.4006 | 0.1784 | 0.1847 | 0.1058 | 0.3434 |
| 14 | -0.4979 | 0.0053 | 0.8604 | -0.3177 | -0.3952 | -0.5313 | -0.4286 | -0.2481 |
| 15 | -0.2229 | -1.0347 | -1.5295 | 0.0698 | 1.5075 | 1.4568 | 1.0163 | -0.5450 |
| 16 | -1.7932 | -0.3768 | -1.0323 | -1.8411 | 0.2447 | -0.0615 | 0.8985 | -0.3783 |
| 17 | -0.9072 | 0.8456 | 0.8276 | 0.8339 | -0.4161 | -0.6761 | -0.8339 | 1.1642 |
| 18 | -1.5284 | -0.1162 | -1.6380 | -0.2464 | 0.9359 | 0.8452 | 1.3838 | 0.3676 |
| 19 | 0.0635 | -0.6466 | 0.2527 | -0.6736 | -0.7311 | -0.7089 | -0.9597 | -0.4938 |
| 20 | -0.3322 | -0.7719 | -0.9688 | -0.5289 | 0.2748 | 0.5604 | -0.1090 | 0.0930 |
| 21 | 0.8363 | -3.1153 | 1.0076 | 0.1893 | -0.1812 | 0.1373 | -0.9965 | -0.9165 |
| 22 | -1.3018 | 0.7665 | -0.5491 | -1.0314 | 1.3271 | 1.3607 | 0.9057 | 0.2032 |
| 23 | 0.8145 | 0.9102 | -0.1833 | 0.3098 | 0.5217 | 0.6273 | 1.2474 | 1.5784 |
| 24 | -1.4599 | -0.3943 | -0.6370 | -0.6204 | 1.7656 | 1.6191 | 1.4431 | -1.1297 |
| 25 | 0.7381 | 1.8522 | 1.2722 | 1.0809 | 1.2301 | 1.6593 | 1.8160 | 0.6969 |
| 26 | -0.0667 | 0.9084 | 0.3115 | -1.2953 | -0.9007 | -0.4908 | -0.4726 | -1.1317 |
| 27 | 0.6741 | 0.5272 | 0.8041 | 1.7265 | -0.7964 | -1.0896 | -1.1621 | -0.6143 |
| 28 | -1.1616 | 0.3207 | -0.6970 | -0.7600 | -1.5502 | -1.3005 | -1.1556 | 0.7772 |
| 29 | -0.2629 | 0.5067 | -0.0460 | 0.1111 | 1.4462 | 0.8822 | 1.5057 | 1.2392 |
| 30 | 1.2883 | 1.1963 | 0.7661 | 0.3061 | 0.2852 | 0.2989 | 1.2586 | 1.1381 |
| 31 | 0.7828 | 0.8571 | 0.1496 | 1.7128 | 0.9654 | 0.9238 | 1.2506 | 2.1281 |
| 32 | 1.5169 | -0.0205 | 0.8464 | -0.0359 | -0.7272 | -0.7278 | -1.2916 | 1.2231 |
| 33 | 0.3509 | -0.6733 | -1.5662 | 1.7560 | 1.1522 | 0.9765 | 1.1164 | 1.1622 |
| 34 | 0.5534 | 1.2369 | 0.0822 | -0.6813 | -0.1755 | -1.1169 | -0.5627 | 0.9485 |
| 35 | -1.0769 | -0.3768 | 0.3173 | -2.2259 | 1.1891 | 0.8828 | 0.9288 | -1.4486 |
| 36 | -1.2112 | -1.4240 | -2.4150 | -0.2752 | -0.6598 | -0.4596 | -1.0491 | -1.5526 |
| 37 | 0.2805 | -0.1312 | 0.4774 | -0.4382 | -0.4866 | -0.2515 | -0.8709 | -0.9360 |
| 38 | 0.0455 | 1.3868 | 0.6457 | 0.6155 | 0.2805 | -0.0781 | 0.1288 | 0.1504 |

(continued)

| No | Pro | Trp | His | LPE (C 18:2) | SM (d18:2, C17:0) | SM (35:1) | SM (41:2) | Cer (d18:1,C24:0) |
|----|------|------|------|------|------|------|------|------|
| 39 | -0.5959 | 0.6325 | 0.6845 | 0.7440 | 0.8471 | 0.9691 | 0.5940 | 1.2250 |
| 40 | -0.0761 | -0.1229 | -0.8475 | -1.0625 | 1.5102 | 0.8790 | 1.3467 | 0.6845 |
| 41 | 0.3298 | -0.4657 | 0.1420 | -0.5257 | -0.2547 | -0.4865 | -0.7028 | 0.9077 |
| 42 | 0.3910 | 1.3778 | 0.6554 | 1.2727 | 0.4169 | 0.1484 | 0.7574 | 0.4749 |
| 43 | -1.0025 | -1.3688 | -2.1767 | -1.0345 | 0.0716 | 0.6633 | 0.1630 | -1.6810 |
| 44 | -0.3425 | -0.9341 | -1.3101 | -0.5431 | -0.8234 | -0.3929 | -1.0100 | -0.8815 |
| 45 | -1.5579 | 0.2579 | -0.8469 | 0.8019 | 2.0422 | 2.0759 | 1.9533 | -0.6812 |
| 46 | -0.3990 | 0.7211 | 0.3805 | -1.0151 | 0.2115 | -0.0431 | -0.0893 | 0.5718 |
| 47 | -0.2508 | -1.0168 | -2.2625 | 0.5614 | -0.7285 | -0.6380 | -0.7118 | 0.3897 |
| 48 | 0.0920 | 1.2886 | 1.1285 | 0.3223 | 0.3704 | 0.0548 | 0.6681 | 1.4049 |
| 49 | 0.6451 | -1.1259 | 0.5793 | 1.3410 | -0.9075 | -0.5203 | -0.3105 | 1.6303 |
| 50 | 0.1377 | -1.1941 | 0.1593 | 0.2136 | -0.0574 | 0.2527 | 0.1012 | 0.8268 |
| 51 | 1.8377 | 0.0791 | -1.2415 | 1.9130 | -1.3957 | -2.1138 | -1.9587 | 1.0433 |
| 52 | 1.3949 | 0.4985 | -0.1585 | -0.0338 | -0.4263 | 0.0750 | 0.2720 | 0.5595 |
| 53 | -0.5621 | -0.0416 | -0.7221 | 0.7862 | -0.8695 | -1.2192 | -1.1711 | -0.0761 |
| 54 | 0.0274 | -0.8775 | -1.0131 | 0.3754 | -1.5979 | -1.4962 | -1.4840 | -1.0625 |
| 55 | 1.9812 | 1.7093 | 0.9392 | 1.4171 | 0.6137 | 0.3569 | 0.0074 | -0.6592 |
| 56 | 0.3036 | -3.9075 | -2.2457 | -1.2155 | -0.4070 | 0.1721 | -1.0411 | -2.0890 |
| 57 | 1.5874 | 1.2839 | 0.5605 | 1.1271 | -1.0639 | -0.7720 | -0.4533 | -0.4750 |
| 58 | 1.7253 | 0.7242 | -0.3538 | 0.3135 | -0.7247 | -0.8666 | -1.1353 | 0.8081 |
| 59 | -0.3384 | 0.2016 | 0.0116 | -0.1685 | 1.0031 | 0.5908 | 1.1364 | 0.7589 |
| 60 | 0.0347 | 0.1780 | -2.1567 | 0.4786 | 0.9647 | 0.8628 | 1.0506 | 1.0844 |
| 61 | 3.4758 | 0.0533 | -1.2891 | 0.3546 | -1.1249 | -0.9725 | -1.0371 | -0.9041 |
| 62 | -0.6904 | 0.0185 | -0.4243 | -0.8386 | -0.6142 | -0.2367 | -0.5943 | 1.1635 |
| 63 | -0.4913 | -0.9590 | 0.4521 | -0.6453 | 0.7224 | 0.5267 | 0.3198 | -2.3544 |
| 64 | 0.6567 | 0.4831 | 0.4404 | 1.9263 | -0.1447 | -0.2535 | -0.2816 | -0.1205 |
| 65 | -0.2569 | 0.4628 | -1.3819 | 0.8796 | 0.4061 | 0.3029 | -0.1588 | -0.0666 |
| 66 | -0.4696 | -0.4834 | -0.4340 | -1.1542 | 0.5359 | 0.5575 | 0.3198 | -0.9324 |
| 67 | -0.6324 | 1.3002 | -0.1625 | -0.7429 | 0.8810 | 0.7622 | 1.1809 | -0.0892 |
| 68 | -0.9250 | 0.6340 | -0.6874 | 0.8458 | -0.3286 | -0.1239 | -0.2453 | -0.2990 |
| 69 | 0.2838 | 0.4401 | -0.1104 | -0.2700 | 1.5083 | 1.0778 | 1.0710 | 1.3847 |
| 70 | 0.3265 | -0.6925 | 0.9895 | 0.5720 | -0.5497 | -1.3466 | -0.9936 | -0.9737 |
| 71 | -0.1660 | -2.3361 | 1.5355 | 0.5157 | -1.0386 | -0.5612 | -2.1956 | 0.3405 |
| 72 | -0.9352 | -0.4036 | -0.5722 | -0.7659 | -0.1345 | 0.2056 | -0.2043 | -0.9901 |
| 73 | 0.4148 | 1.6552 | 1.0122 | 1.5196 | 0.3016 | -0.3794 | 1.0214 | 0.9682 |
| 74 | -0.7640 | -0.6568 | -1.0537 | 0.1583 | -0.0280 | 0.5385 | 0.2213 | 0.0283 |
| 75 | 0.6320 | 0.7939 | 0.4582 | 0.0539 | -0.4583 | -0.2200 | -0.1164 | 0.4923 |
| 76 | -0.7377 | -1.0623 | -1.4339 | -0.8871 | -1.6257 | -1.4195 | -1.5635 | -1.7327 |
| 77 | 0.9101 | 1.2107 | -0.0572 | 1.3519 | 0.6160 | 0.4971 | 0.6912 | 1.2263 |

(continued)

| No | Pro | Trp | His | LPE (C 18:2) | SM (d18:2, C17:0) | SM (35:1) | SM (41:2) | Cer (d18:1,C24:0) |
|---|---|---|---|---|---|---|---|---|
| 78 | 0.4100 | -0.4571 | 0.6603 | 0.5419 | 0.0608 | 0.0371 | 0.2589 | 0.7915 |
| 79 | -0.2892 | 0.3886 | -0.5897 | -1.2604 | 3.7637 | 3.8402 | 3.4545 | 1.1063 |
| 80 | -0.4159 | 0.3221 | -0.8495 | 1.2350 | -0.3286 | -0.6610 | -0.3476 | 1.2930 |
| 81 | -2.2840 | -1.7830 | -1.8526 | 0.2103 | -2.4088 | -2.2057 | -1.7668 | 0.1235 |
| 82 | 0.0056 | 0.4957 | 1.9675 | 0.9386 | -1.1108 | -1.0611 | -0.6466 | 0.9820 |
| 83 | -0.7833 | 0.4619 | 0.6114 | 0.2371 | 1.2177 | 0.8090 | 1.5189 | 0.6160 |
| 84 | -1.0769 | 0.9492 | 0.4363 | -0.0734 | 0.6751 | 0.8229 | 0.6313 | -0.3783 |
| 85 | -0.6531 | -0.9460 | 0.3188 | 0.3967 | -0.2976 | -0.1581 | -0.8981 | -0.1771 |
| 86 | 0.4695 | -0.4774 | 1.1725 | 1.3886 | 0.3136 | 0.5495 | 0.5999 | 1.7641 |
| 87 | -0.8199 | -0.8206 | -1.2279 | -0.5431 | 0.5034 | 0.5872 | 0.3219 | -0.6343 |
| 88 | 0.3734 | 1.3416 | 1.5110 | 0.2501 | -1.3418 | -0.6437 | -0.7178 | 0.1332 |
| 89 | -0.2750 | 0.9086 | 1.0257 | 1.0286 | -0.4942 | -1.4740 | -0.5174 | 0.6272 |
| 90 | 1.6754 | -0.3908 | 1.4161 | 0.0913 | -1.1782 | -1.1487 | -1.6399 | -0.9235 |
| 91 | 0.1723 | -0.8738 | 0.4536 | -0.4569 | 0.7293 | 0.7207 | 0.8931 | -0.2623 |
| 92 | -1.2219 | -0.8108 | 0.4521 | -1.9405 | -0.8017 | -0.9299 | -0.8029 | -1.9287 |
| 93 | -0.5088 | 0.3371 | -0.0578 | -1.7197 | 0.0485 | -0.1821 | 0.3413 | -0.7018 |
| 94 | -0.4308 | -1.0936 | -1.4138 | -0.3549 | 0.8352 | 1.3391 | 0.8679 | -0.5764 |
| 95 | 0.0165 | -1.3090 | -0.0202 | -0.0860 | 0.4251 | -0.0523 | 0.3284 | -0.3976 |
| 96 | 0.8781 | 0.6768 | 0.3846 | 0.5030 | -0.1168 | -0.0167 | 0.0098 | 1.0058 |
| 97 | 0.0973 | 2.1371 | 1.2550 | 2.2937 | -0.3308 | -0.4596 | 0.1788 | 1.9696 |
| 98 | -0.2269 | 0.4491 | 0.5764 | 0.4478 | 0.1140 | -0.0596 | 0.6117 | 0.9249 |
| 99 | -0.1293 | 0.3194 | 0.4688 | 0.0016 | -1.0008 | -0.4181 | -0.2247 | 0.3792 |
| 100 | 0.0092 | 0.8048 | 1.3868 | -0.0122 | -0.0456 | -0.2220 | 0.1697 | -0.5930 |
| 101 | -0.9224 | 0.7051 | -2.2352 | 0.3058 | -0.9754 | -0.3310 | -0.6790 | -0.4880 |
| 102 | -0.9480 | -1.0879 | 0.4435 | -1.8564 | 0.8731 | 1.0068 | 0.8931 | -1.6063 |
| 103 | -0.6049 | -0.1766 | -0.8761 | -1.6295 | -0.4508 | -0.3035 | -0.2764 | 0.5185 |
| 104 | -0.7329 | -0.3690 | 0.6700 | -1.2756 | 0.5311 | 0.2957 | 0.3025 | -0.8351 |
| 105 | 0.6899 | 0.5188 | 1.3447 | 0.0776 | -1.0603 | -1.2828 | -0.9134 | -0.6112 |
| 106 | -0.2549 | -0.1508 | 0.7423 | 0.8772 | -0.5295 | -0.2377 | -0.2145 | 0.6770 |
| 107 | 1.1142 | -1.4593 | -0.5922 | -0.0994 | -0.0421 | -0.1610 | -0.1638 | 0.1450 |
| 108 | 0.5878 | -1.2421 | 0.2262 | 0.9970 | -0.2525 | -0.1783 | -0.3343 | 0.8821 |
| 109 | 0.6408 | 0.1066 | 1.4864 | 0.7037 | -1.4381 | -1.5932 | -1.2891 | -0.0832 |
| 110 | -0.4891 | -0.3159 | 0.8698 | 0.6265 | -1.2095 | -0.8504 | -0.5770 | 1.0857 |
| 111 | -0.7045 | 0.6373 | 0.4850 | 0.3084 | 0.4185 | 0.9155 | 0.6527 | -0.2136 |
| 112 | 0.4880 | 0.9987 | -0.4207 | 0.2112 | 0.7004 | 0.9734 | 0.8370 | 0.1833 |
| 113 | -0.0036 | -2.0579 | -1.1668 | 0.1072 | -1.7201 | -1.8164 | -1.6495 | -0.3811 |
| 114 | -0.5913 | 0.4134 | 0.7566 | -1.5826 | 0.3094 | 0.0130 | 0.1196 | -1.1336 |
| 115 | -0.3633 | -0.4107 | 0.3021 | 0.8288 | -1.0250 | -0.9273 | -1.1473 | -0.1659 |
| 116 | -0.5509 | -0.2447 | -0.1729 | -0.5784 | 0.0031 | 0.3356 | 0.9606 | -0.7666 |

(continued)

| No | Pro | Trp | His | LPE (C 18:2) | SM (d18:2, C17:0) | SM (35:1) | SM (41:2) | Cer (d18:1,C24:0) |
|----|------|------|------|------|------|------|------|------|
| 117 | 0.1844 | -0.1685 | -0.0409 | 0.7673 | 0.1218 | 0.3459 | 0.4215 | 0.5185 |
| 118 | -1.1094 | -0.0312 | -0.2525 | -2.2387 | -0.3303 | -0.3198 | -0.0069 | -1.3777 |
| 119 | -0.2790 | -0.7867 | 0.2843 | -1.3729 | -1.2513 | -1.3406 | -1.6387 | -0.7848 |
| 120 | 3.0863 | -1.6096 | 1.7426 | 1.5238 | 0.6804 | 0.2354 | 1.3960 | 1.7108 |
| 121 | -0.3363 | -0.3215 | 0.7375 | -1.3213 | -0.5875 | -0.4822 | 0.1379 | -1.0569 |
| 122 | 0.1568 | 1.5244 | 0.5116 | 0.9840 | -0.1376 | 0.2131 | 0.0687 | 1.0594 |
| 123 | -1.2293 | -1.7579 | -1.6155 | -0.7778 | 0.4875 | 0.8242 | -0.2973 | -0.7082 |
| 124 | -2.1375 | 0.2565 | -0.6472 | -0.2656 | 2.3241 | 2.2984 | 1.9043 | -0.8522 |
| 125 | -1.1915 | 0.7597 | 0.0027 | -1.9665 | 1.0954 | 1.1152 | 0.8443 | -1.9013 |
| 126 | -0.6163 | -1.2665 | 0.1894 | -1.0587 | 1.6808 | 1.6660 | 0.9412 | -0.6827 |
| 127 | -1.2879 | -0.1762 | -1.6387 | -1.3296 | -0.0594 | 0.5149 | 0.9588 | -0.6250 |
| 128 | -0.1045 | -1.3542 | -1.9089 | -0.0842 | 0.6061 | 1.3848 | 0.2698 | -1.0475 |
| 129 | -0.5066 | 0.1381 | -0.2654 | -0.9016 | 0.4467 | 1.0025 | 1.0007 | 0.0974 |
| 130 | -1.1561 | 0.5578 | 1.4369 | 0.7786 | -0.2372 | 0.1373 | 0.6507 | 0.3030 |
| 131 | 0.0056 | -0.2381 | -0.2537 | -0.7155 | 0.4613 | 0.6038 | 0.6235 | 0.7572 |
| 132 | 0.1429 | -0.7669 | 0.8182 | 0.8104 | -0.8628 | -0.6518 | -0.1164 | 1.1138 |
| 133 | 1.2299 | 1.1745 | 1.5881 | 1.6594 | -0.1366 | 0.0121 | 0.4090 | 1.7062 |
| 134 | -0.6027 | 0.2720 | 0.4835 | 1.0120 | -0.7279 | -1.7614 | -2.3828 | 0.2608 |
| 135 | 1.5126 | 0.8204 | -0.2943 | 0.7373 | -1.5450 | -1.6113 | -1.5051 | 0.8631 |
| 136 | -0.2368 | 0.4366 | 0.8651 | 1.2564 | -0.2922 | -0.8716 | -0.2376 | 1.0157 |
| 137 | 4.2929 | -0.5712 | 1.3531 | 1.0887 | -1.1563 | -1.3965 | -1.7723 | 0.3888 |
| 138 | 0.9140 | 1.2322 | 0.7899 | -0.3613 | -0.6002 | -0.6380 | -0.3770 | 0.5586 |
| 139 | -0.6049 | 0.8887 | 1.1417 | 1.0842 | 0.7399 | 0.8803 | 0.8697 | 1.7560 |
| 140 | 0.0092 | 0.9089 | 2.0157 | 1.3488 | 0.6729 | 0.4595 | 1.0930 | 0.9639 |
| 141 | -1.2673 | -0.3104 | -0.9940 | -0.5261 | -0.9096 | -0.5556 | -1.1394 | 0.1289 |
| 142 | -0.8471 | -0.0140 | -0.8747 | -0.4763 | 0.4686 | 0.5575 | 0.5059 | 0.2486 |
| 143 | 0.9560 | 0.3971 | -0.6061 | 0.1101 | -1.5120 | -1.8344 | -1.3278 | -0.2148 |
| 144 | 0.5308 | 0.0260 | 0.6457 | -0.1560 | -2.0312 | -1.8182 | -1.6294 | -0.8317 |
| 145 | 1.0343 | 0.9752 | 0.3345 | -0.3668 | -0.7645 | -0.4436 | -0.5827 | 0.1864 |
| 146 | 1.2002 | 0.9935 | 1.3192 | -1.1650 | -1.1465 | -1.9224 | -1.2819 | -0.5465 |
| 147 | 0.1061 | -0.1177 | 0.5001 | 0.6946 | 0.2288 | -0.1155 | 0.0547 | -0.0928 |
| 148 | 0.3085 | 0.5955 | -0.2059 | -0.6594 | 0.9077 | 0.9709 | -0.5456 | -0.4508 |
| 149 | -0.7858 | -0.0980 | -0.0235 | -2.5944 | -1.3177 | -1.1432 | -0.6761 | -1.0214 |
| 150 | -0.1855 | -0.4102 | 0.0371 | -0.4700 | -0.3550 | -0.5346 | -0.4865 | -0.8607 |
| 151 | -0.3863 | 0.4815 | 0.3547 | 0.9892 | 0.9396 | 0.5815 | 0.5140 | 0.5988 |
| 152 | 0.6784 | -0.5099 | -1.7298 | -0.7702 | 0.7381 | 0.4701 | -0.2790 | -1.1084 |
| 153 | -0.6857 | 1.0931 | -0.9397 | -0.5173 | 0.6594 | 0.8803 | 0.5201 | 0.2770 |
| 154 | 0.7212 | -0.3707 | -1.8942 | 0.2127 | 0.0074 | -0.4160 | 0.0989 | 0.3010 |
| 155 | -0.0931 | -0.9062 | 0.4006 | -0.7265 | 0.4032 | -0.2064 | -0.7148 | -1.1142 |

(continued)

| No | Pro | Trp | His | LPE (C 18:2) | SM (d18:2, C17:0) | SM (35:1) | SM (41:2) | Cer (d18:1,C24:0) |
|---|---|---|---|---|---|---|---|---|
| 156 | -1.2740 | -0.8539 | -0.9763 | -2.4401 | -0.3890 | 0.1535 | -0.0117 | -1.6284 |

Table 5: Coefficient values for the i-metabolic signature in th emodel according to Mahaj an et al. 2022; two biomarkers (Sphingomyelin (41:2) and Ceramide (d18:1,C24:0)) were excluded from the model due to regularization.

| Coefficient_Order | Coefficient | Variable | Variable Name |
|---|---|---|---|
| β1 | 1.4126 | X1 | CA19.9 |
| β2 | -0.4391 | X2 | Proline |
| β3 | -0.1511 | X3 | Tryptophan |
| β4 | -0.2891 | X4 | Histidine |
| β5 | -0.4655 | X5 | Lysophosphatidylethanolamine (C18:0) |
| β6 | -0.0241 | X6 | Lysophosphatidylethanolamine (C18:2) |
| β7 | 0.3730 | X7 | Sphingomyelin (d17:1,C16:0) |
| β8 | 0.0793 | X8 | Sphingomyelin (d18:2,C17:0) |
| β9 | 0.3628 | X9 | Sphingomyelin (35:1) |
| β10 | | X10 | Sphingomyelin (41:2) |
| β11 | 0.5053 | X11 | Phosphatidylethanolamine (d18:0,C22:6) |
| β12 | -0.2705 | X12 | Ceramide (d18:2,C24:0) |
| β13 | | X13 | Ceramide (d18:1,C24:0) |

Table 6: Performance marker comparison

| N = (PDAC /Total) | Model | AUC | DeLong's test[1] [Z (P)] | Sensitivity | Specificity | Balanced accuracy |
|---|---|---|---|---|---|---|
| | i-Metabolic signature (present invention) | 0.846 (0.842 - 0.849) | Z = 21.417 P <0.001 | 0.675 (0.669 - 0.680) | 0.904 (0.898 - 0.911) | 0.805 (0.802- 0.808) |
| All Stages (N = 489) | m-Metabolic signature (present invention) | 0.846 (0.842 - 0.849) | Z = 21.417 P <0.001 | 0.599 (0.593 - 0.604) | 0.936 (0.931 - 0.940) | 0.790 (0.788 - 0.792) |
| | CA19.9 Alone | 0.799 (0.797 - 0.802) | | 0.818 (0.815 - 0.820) | 0.791 (0.787 - 0.794) | 0.806 (0.804 - 0.809) |
| | i-Metabolic signature (Mahajan et al., 2022) | 0.481 (0.478 - 0.484) | Z = -160.000 P <0.001 | 0.561 (0.556 - 0.565) | 0.428 (0.423 - 0.732) | 0.498 (0.496 - 0.501) |

**Claims**

1. A method for assessing pancreatic cancer in a subject, said method comprising

(a) determining the biomarkers Ceramide (d18: 1;C24:0), Lysophosphatidylethanolamine (C18:0), Phosphatidylethanolamine (C18:0, C22:6), Sphingomyelin (d17:1; C16:0), and CA19.9 in a sample from said subject;
(b) comparing the biomarkers determined in step (a) to at least one corresponding reference; and
(c) assessing pancreatic cancer in said subject based on said comparing in step (b).

2. The method of claim 1, wherein assessing comprises excluding pancreatic cancer, preferably identifying a subject not suffering from pancreatic cancer; diagnosing pancreatic cancer, preferably diagnosing PDAC; staging pancreatic cancer; prognosticating pancreatic cancer; and/or differentiating between pancreatic cancer and a non-pancreatic cancer disorder, preferably pancreatitis, more preferably chronic pancreatitis.

3. The method of claim 1 or 2, wherein said subject is a subject at risk of suffering from pancreatic cancer; preferably is known or suspected to suffer from new onset type 2 diabetes, chronic pancreatitis, pancreatic cystic lesions, and/or is from a familial PDAC kinship.

4. The method of any one of claims 1 to 3, wherein said method comprises further determining at least one, preferably at least two, more preferably at least three, even more preferably at least four, most preferably all, marker(s) selected from the list consisting of Histidine, Proline, Tryptophan, Ceramide (d18:2,C24:0), Lysophosphatidylethanolamine (C18:2), Sphingomyelin (35:1), Sphingomyelin (41:2), and Sphingomyelin (d18:2,C17:0), preferably in step (a).

5. The method of any one of claims 1 to 4, wherein said sample is a bodily fluid sample, preferably a blood sample or a blood-derived sample, more preferably a blood, plasma, or serum sample, even more preferably a plasma or serum sample.

6. The method of any one of claims 1 to 5, wherein step (b) comprises calculating a predictor score for assessing pancreatic cancer from all of said biomarkers and wherein said predictor score is calculated according to formula (I)

$$P = \frac{1}{1 + e^{-z}}$$

wherein P= predictor score; and $z = \beta_0 + \beta_1 X_1 + \beta_2 X_2 + \cdots + \beta_n X_n$
and wherein variables $X_1, X_2, ..., X_n$ are measurement values of said biomarkers.

7. The method of claim 6, wherein coefficients $\beta_0, \beta_1, \beta_2, ..., \beta_n$ are selected from Table 1 or from Table 2.

8. The method of any one of claims 1 to 7, wherein said method comprises determining at least one further biomarker, preferably selected from the list consisting of aspartate aminotransferase, alanine aminotransferase, platelet count, haptoglobin, alpha2-macroglobulin, apolipoprotein A1, bilirubin, cholesterol, hyaluronan, prothrombin index, hepatocyte growth factor (HGF), Tissue inhibitor of metalloproteinases (TIMP), and urea; and/or wherein said method comprises further diagnostic steps, preferably sonography, magnetic resonance imaging, radiography, transient elastography, and/or determining subject age and/or gender.

9. The method of any one of claims 1 to 8, wherein said method is computer-implemented, preferably wherein said method is computer implemented and wherein said comparing in step (b) is performed by a trained automated machine learning derived generalized logistic regression model, more preferably wherein said logistic regression model was trained as specified in any one of claims 10 to 13.

10. A computer-implemented training method of training at least one trainable model for assessing pancreas cancer in a subject, the method comprising:

(i) providing the trainable model;
(ii) retrieving labeled training subject data comprising biomarker data of subjects having known pancreas cancer states, wherein said biomarker data are data of the biomarkers Ceramide (d18:1;C24:0), Lysophosphatidylethanolamine (C18:0), Phosphatidylethanolamine (C18:0, C22:6), Sphingomyelin (d17: 1; C16:0), and CA19.9; and
(iii) training the trainable model on the labeled training subject data,

wherein said trainable model is a Generalized Linear Model (GLM).

**11.** The method of claim 10, wherein said assessing is classifying pancreas cancer.

**12.** The method of claim 10 or 11, wherein the trainable model is a binomial GLM.

**13.** The method of any one of claims 10 to 12, wherein the trainable GLM model is trained using the following hyperparameters:

Regularization: Ridge;
Logistic regression link: logit;
Number of iterations: 40,
$N_{lambad}$ = 30,
Lambda.max = 32.331
Sort_metric = "F1", and/or
Stopping tolerance = 3.

**14.** An automated machine learning model obtained or obtainable according to the method according to any one of claims 10 to 13, preferably tangibly embedded on a data storage means.

**15.** A system comprising

(i) a measuring device for determining the biomarkers Ceramide (d18:1;C24:0), Lysophosphatidylethanolamine (C18:0), Phosphatidylethanolamine (C18:0, C22:6), Sphingomyelin (d17: 1; C16:0), and CA19.9; and
(ii) an evaluation device operably linked to the measuring device, said evaluation device comprising a data processor comprising instructions for carrying out a comparison of the biomarkers determined by the measuring device to at least one corresponding reference, wherein said system is adapted to perform the method according to any one of claims 1 to 9, preferably comprising tangibly embedded instructions which, when carried out by the data processor, cause the system to perform a method according to any one of claims 1 to 9.

## Figure 1

**A** Cohort enrichment design — Patients enrollment

Effect size / Sample size

General population (~0.0135% risk)
Sample size >800,000 cases

Patients with High-risk of Pancreatic cancer (~1% risk)
Sample size ~29000 cases

Patients with Undefined mass in Pancreas on diagnostic imaging (~20% risk)
Sample size ~1375 cases
● PDAC
● Others*

**B** METAPAC

**1370** Patients with undefined mass in pancreas approached for consent

**17** Excluded
  **3** Inclusion criteria not met
  **10** Consent withdrawn
  **4** Others

**1353** Patients enrolled

**40** Dropouts
  **10** Failed phlebotomy
  **2** Sample outage
  **15** Insufficient/degraded plasma
  **13** Others

**1313** Underwent targeted metabolome analysis

**184** Dropouts
  **125** Missing CA19.9 values
  **59** Not confirmed pancreas specific etiology

**1129** Included in primary analysis
  **489** PDAC
  **232** IPMNs
  **271** Non-IPMNs Cystic lesions
  **113** Chronic pancreatitis
  **11** Acute pancreatitis
  **13** Metastases of extra-pancreatic origin

**C**

Hamburg, Greifswald, Berlin, Hannover, Gehrden, Bochum, Halle, Leipzig, Jena, Dresden, Gera, Marburg, Würzburg, Erlangen, Heidelberg, Ulm, Freiburg, Munich

Number of Patients ○ 100 ● 200 ● 300 ● 400

**D**

### Population vs Enriched Cohort Prevalence
$R^2 = 0.834$, $P < 0.001$

Cohort Prevalence (%) vs Population Prevalence (%)

PDAC, Cystic lesions including IPMNs, Smoking, Age (Median Age), Diabetes Mellitus, BMI, Chronic Pancreatitis, Relatives with PDAC, Gender (M/F Ratio)

○ Primary endpoint
○ risk factors
● risk groups

**E**

| Metabolites | i-Metabolite signature | m-Metabolite signature |
|---|---|---|
| CA19.9 | X | X |
| Histidine | X | - |
| Proline | X | - |
| Tryptophan | X | - |
| Ceramide (d18:1,C24:0) | X | X |
| Ceramide (d18:2,C24:0) | X | - |
| Lysophosphatidylethanolamine (C18:0) | X | X |
| Lysophosphatidylethanolamine (C18:2) | X | - |
| Phosphatidylethanolamine (C18:0,C22:6) | X | X |
| Sphingomyelin (d17:1,C16:0) | X | X |
| Sphingomyelin (35:1) | X | - |
| Sphingomyelin (41:2) | X | - |
| Sphingomyelin (d18:2,C17:0) | X | - |
| Cut-off | 0.676 | 0.773 |

## Figure 2

## Figure 3

A

SHIP-TREND-0
2008-2012
(n = 4420)

5-yr follow-up

SHIP-TREND-1
2016-2019
(n = 2507)

**243** New Onset Diabetes
Mellitus (NOD)
(**3** Pancreatic cancer (PDAC))

**243** Underwent un-targeted
metabolome analysis

**242** Included in analysis

B

*Performance of m–Metabolite signature*

*Without CA19.9*

P = 0.038

○ Underweight
○ Normal
○ Overweight
○ Obese

26.01
29.73

NODM  PDAC

**EP 4 786 983 A1**

<table>
<tr><td colspan="4" align="center">**EUROPEAN SEARCH REPORT**</td><td>**Application Number**<br>EP 25 15 5667</td></tr>
</table>

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | MAHAJAN UJJWAL M ET AL: "Independent Validation and Assay Standardization of Improved Metabolic Biomarker Signature to Differentiate Pancreatic Ductal Adenocarcinoma From Chronic Pancreatitis", GASTROENTEROLOGY (AUTHOR MANUSCRIPT), W.B. SAUNDERS, AMSTERDAM, NL, vol. 163, no. 5, 21 July 2022 (2022-07-21), pages 1407-1422, XP087204360, ISSN: 0016-5085, DOI: 10.1053/J.GASTRO.2022.07.047 [retrieved on 2022-07-21] | 1-3,5-15 | INV.<br>G01N33/574<br>G01N33/92<br>G16B40/00<br>G16H50/00 |
| Y | * figures 1, 3B *<br>* the whole document *<br>* Results *<br>* Conclusion * | 4 | |
| Y | MAYERLE JULIA ET AL: "Metabolic biomarker signature to differentiate pancreatic ductal adenocarcinoma from chronic pancreatitis", GUT MICROBIOTA, vol. 67, no. 1, 20 January 2017 (2017-01-20), pages 128-137, XP093289903, UK ISSN: 0017-5749, DOI: 10.1136/gutjnl-2016-312432<br>* table 1 *<br>* the whole document * | 4 | **TECHNICAL FIELDS SEARCHED (IPC)**<br>G01N<br>G06F<br>G16H<br>G16B |

The present search report has been drawn up for all claims

| Place of search | Date of completion of the search | Examiner |
|---|---|---|
| The Hague | 3 July 2025 | Biegler, Marcel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

42

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KIM et al.** *Sci Rep*, 2023, vol. 13 (1), 106
- **SINGHI et al.** *Gastroenterology*, 2019, vol. 156 (7), 2024-40
- **LLACH et al.** *Cancer Manag Res*, 2020, vol. 12, 743-58
- **MAYERLE et al.** *Gut*, 2018, vol. 67 (1), 128-37
- **PEPE et al.** *J Natl Cancer*, 2008, vol. 100 (20), 1432-8
- **MAHAJAN et al.** *Gastroenterology*, 2022, vol. 163 (5), 1407-22
- **DOWDY** ; **WEARDEN**. Statistics for Research. John Wiley & Sons, 1983
- **BRAND et al.** *Gut.*, 2007, vol. 56, 1460-9
- **DEL CHIARO et al.** *World J Gastroenterol*, 2014, vol. 20, 12118-12131
- *CHEMICAL ABSTRACTS*, 102917-80-6
- *CHEMICAL ABSTRACTS*, 899443-67-5
- *CHEMICAL ABSTRACTS*, 202647-82-3
- *CHEMICAL ABSTRACTS*, 123065-40-7
- *CHEMICAL ABSTRACTS*, 92448-22-1
- *CHEMICAL ABSTRACTS*, 71-00-1
- *CHEMICAL ABSTRACTS*, 147-85-3
- *CHEMICAL ABSTRACTS*, 73-22-3
- *CHEMICAL ABSTRACTS*, 135941
- *CHEMICAL ABSTRACTS*, 85046-18-0
- *CHEMICAL ABSTRACTS*, 635-65-4
- *CHEMICAL ABSTRACTS*, 57-88-5
- *CHEMICAL ABSTRACTS*, 9004-61-9
- *CHEMICAL ABSTRACTS*, 57-13-6
- **ZWEIG**. *Clin. Chem.*, 1993, vol. 39, 561-577
- **BEN-AMI** ; **RONI** ; **QIAO-LI WANG** ; **JINMING ZHANG** ; **JULIANNA G SUPPLEE** ; **JOHANNES F FAHRMANN** ; **RONI LEHMANN-WERMAN** ; **LAUREN K BRAIS et al.** Protein Biomarkers and Alternatively Methylated Cell-Free DNA Detect Early Stage Pancreatic Cancer. *Gut*, 2023
- **BOYD** ; **LENKA N. C.** ; **MAHSOEM ALI** ; **ANNALISA COMANDATORE** ; **INGRID GARAJOVA** ; **LAURA KAM** ; **JISCE R. PUIK** ; **STEPHANIE M. FRAGA RODRIGUES et al.** Prediction Model for Early-Stage Pancreatic Cancer Using Routinely Measured Blood Biomarkers. *JAMA Network Open*, 2023, vol. 6 (8), e2331197
- **BRAND, RANDALL E.** ; **JAN PERSSON** ; **SVEIN OLAV BRATLIE** ; **DANIEL C. CHUNG** ; **BRYSON W. KATONA** ; **ALFREDO CARRATO** ; **MARIÉN CASTILLO et al.** Detection of Early-Stage Pancreatic Ductal Adenocarcinoma From Blood Samples: Results of a Multiplex Biomarker Signature Validation Study. *Clinical and Translational Gastroenterology*, 2022, vol. 13 (3)
- **BYEON** ; **SOOIN** ; **MATTHEW J. MCKAY** ; **MARK P. MOLLOY** ; **ANTHONY J. GILL** ; **JASWINDER S. SAMRA** ; **ANUBHAV MITTAL** ; **SUMIT SAHNI**. Novel Serum Protein Biomarker Panel for Early Diagnosis of Pancreatic Cancer. *International Journal of Cancer*, 2024, vol. 155 (2), 365-371
- **CAO** ; **YINGYING** ; **RUI ZHAO** ; **KAI GUO** ; **SHUAI REN** ; **YAPING ZHANG** ; **ZIPENG LU** ; **LEI TIAN** ; **TAO LI** ; **XIAO CHEN**. Potential Metabolite Biomarkers for Early Detection of Stage-I Pancreatic Ductal Adenocarcinoma. *Frontiers in Oncology*, 2022, vol. 11, 744667
- **COHEN, JÉRÉMIE F.** ; **DANIËL A. KOREVAAR** ; **DOUGLAS G. ALTMAN** ; **DAVID E. BRUNS** ; **CONSTANTINE A. GATSONIS** ; **LOTTY HOOFT** ; **LES IRWIG et al.** STARD 2015 Guidelines for Reporting Diagnostic Accuracy Studies: Explanation and Elaboration. *BMJ Open*, 2016, vol. 6 (11), e012799
- **COLLINS, GARY S** ; **JOHANNES B REITSMA** ; **DOUGLAS G ALTMAN** ; **KAREL MOONS**. Transparent Reporting of a Multivariable Prediction Model for Individual Prognosis or Diagnosis (TRIPOD): The TRIPOD Statement. *BMC Medicine*, 2015, vol. 13 (1), 1
- **DIGIACOMO** ; **LUCA** ; **ERICA QUAGLIARINI** ; **DANIELA POZZI** ; **ROBERTO COPPOLA** ; **GIULIO CARACCIOLO** ; **DAMIANO CAPUTO**. Stratifying Risk for Pancreatic Cancer by Multiplexed Blood Test. *Cancers*, 2023, vol. 15 (11), 2983
- **FAHRMANN, JOHANNES F.** ; **C. MAX SCHMIDT** ; **XIANGYING MAO** ; **EHSAN IRAJIZAD** ; **MAUREEN LOFTUS** ; **JINMING ZHANG** ; **NIKUL PATEL et al.** Lead-Time Trajectory of CA19-9 as an Anchor Marker for Pancreatic Cancer Early Detection. *Gastroenterology*, 2021, vol. 160 (4), 1373-1383

- **FIRPO, MATTHEW A.** ; **KENNETH M. BOUCHER** ; **JOSH BLEICHER** ; **GAYATRI D. KHANDERAO** ; **ALESSANDRA ROSATI** ; **KATHERINE E. PORUK** ; **SAMA KAMAL et al.** Multianalyte Serum Biomarker Panel for Early Detection of Pancreatic Adenocarcinoma. *JCO Clinical Cancer Informatics*, 2023 (7), e2200160
- **HAAB** ; **BRIAN** ; **LU QIAN** ; **BEN STAAL** ; **MANEESH JAIN** ; **JOHANNES FAHRMANN** ; **CHRISTINE WORTHINGTON** ; **DENISE PROSSER et al.** A Rigorous Multi-Laboratory Study of Known PDAC Biomarkers Identifies Increased Sensitivity and Specificity over CA19-9 Alone. *Cancer Letters*, 2024, vol. 604, 217245
- **IWANO** ; **TOMOHIKO** ; **KENTARO YOSHIMURA** ; **GENKI WATANABE** ; **RYO SAITO** ; **SHO KIRITANI** ; **HIROMICHI KAWAIDA** ; **TAKESHI MORIGUCHI et al.** High-Performance Collective Biomarker from Liquid Biopsy for Diagnosis of Pancreatic Cancer Based on Mass Spectrometry and Machine Learning. *Journal of Cancer*, 2021, vol. 12 (24), 7477-87
- **KATONA, BRYSON W.** ; **CHRISTINE WORTHINGTON** ; **DANIEL CLAY** ; **HANNAH CINCOTTA** ; **NUZHAT A. AHMAD** ; **GREGORY G. GINSBERG** ; **MICHAEL L. KOCHMAN** ; **RANDALL E. BRAND**. Outcomes of the IMMray PanCan-d Test in High-Risk Individuals Undergoing Pancreatic Surveillance: Pragmatic Data and Lessons Learned. *JCO Precision Oncology*, 2023 (7), e2300445
- **KIM** ; **YOSEOP** ; **INJOON YEO** ; **IKSOO HUH** ; **JAENYEON KIM** ; **DOHYUN HAN** ; **JIN-YOUNG JANG** ; **YOUNGSOO KIM**. Development and Multiple Validation of the Protein Multi-Marker Panel for Diagnosis of Pancreatic Cancer. *Clinical Cancer Research*, 2021, vol. 27 (8), 2236-45
- **LI, TIANDONG** ; **JUNFEN XIA** ; **HUAN YUN** ; **GUIYING SUN** ; **YAJING SHEN** ; **PENG WANG** ; **JIANXIANG SHI** ; **KEYAN WANG** ; **HONGWEI YANG** ; **HUA YE**. A Novel Autoantibody Signatures for Enhanced Clinical Diagnosis of Pancreatic Ductal Adenocarcinoma. *Cancer Cell International*, 2023, vol. 23 (1), 273
- **MAHAJAN, UJJWAL M.** ; **BETTINA OEHRLE** ; **SIMON SIRTL** ; **AHMED ALNATSHA** ; **ELISABETTA GONI** ; **IVONNE REGEL** ; **GEORG BEYER et al.** Independent Validation and Assay Standardization of Improved Metabolic Biomarker Signature to Differentiate Pancreatic Ductal Adenocarcinoma From Chronic Pancreatitis. *Gastroenterology*, 2022, vol. 163 (5), 1407-22
- **MAYERLE** ; **JULIA** ; **HOLGER KALTHOFF** ; **REGINA RESZKA** ; **BEATE KAMLAGE** ; **ERIK PETER** ; **BODO SCHNIEWIND** ; **SANDRA GONZÁLEZ MALDONADO et al.** Metabolic Biomarker Signature to Differentiate Pancreatic Ductal Adenocarcinoma from Chronic Pancreatitis. *Gut*, 2018, vol. 67 (1), 128-37
- **NAKAMURA** ; **KOTA** ; **ZHONGXU ZHU** ; **SOUVICK ROY** ; **EUNSUNG JUN** ; **HAIYONG HAN** ; **RUBEN M. MUNOZ** ; **SATOSHI NISHIWADA et al.** An Exosome-Based Transcriptomic Signature for Noninvasive, Early Detection of Patients With Pancreatic Ductal Adenocarcinoma: A Multicenter Cohort Study. *Gastroenterology*, 2022, vol. 163 (5), 1252-1266
- **PEPE et al.** *J Natl Cancer Inst*, 2008, vol. 100 (20), 1432-8
- **SAHNI** ; **SUMIT** ; **ADVAIT R. PANDYA** ; **WILLIAM J. HADDEN** ; **CHRISTOPHER B. NAHM** ; **SARAH MALONEY** ; **VICTORIA COOK** ; **JAMES A. TOFT et al.** A Unique Urinary Metabolomic Signature for the Detection of Pancreatic Ductal Adenocarcinoma. *International Journal of Cancer*, 2021, vol. 148 (6), 1508-18
- **SEEGER** ; **NICO** ; **STEFAN GUTKNECHT** ; **IRIN ZSCHOKKE** ; **ISABELLA FLEISCHMANN** ; **NADJA ROTH** ; **JÜRG METZGER** ; **MARKUS WEBER** ; **STEFAN BREITENSTEIN** ; **LUKASZ FILIP GROCHOLA**. A Predictive Noninvasive Single-Nucleotide Variation-Based Biomarker Signature for Resectable Pancreatic Cancer: Protocol for a Prospective Validation Study. *JMIR Research Protocols*, 2024, vol. 13, e54042
- Leveraging Genomic Signatures of Oral Microbiome-Associated Antibiotic Resistance Genes for Diagnosing Pancreatic Cancer. **SHEN** ; **XIAOJING** ; **XIAOLIN ZHU** ; **HAIRONG LIU** ; **RONGTAO YUAN** ; **QINGYUAN GUO** ; **PENG ZHAO**. PLOS ONE. 2024, vol. 19, e0302361
- **SONG, JIN** ; **LORI J. SOKOLL** ; **DANIEL W. CHAN** ; **ZHEN ZHANG**. Validation of Serum Biomarkers That Complement CA19-9 in Detecting Early Pancreatic Cancer Using Electrochemiluminescent-Based Multiplex Immunoassays. *Biomedicines*, 2021, vol. 9 (12), 1897
- **SRIVASTAVA** ; **SUDHIR** ; **PAUL D. WAGNER**. The Early Detection Research Network: A National Infrastructure to Support the Discovery, Development, and Validation of Cancer Biomarkers. *Cancer Epidemiology, Biomarkers & Prevention: A Publication of the American Association for Cancer Research, Cosponsored by the American Society of Preventive Oncology*, 2020, vol. 29 (12), 2401-10
- **WEN** ; **YAN-RONG** ; **XIA-WEN LIN** ; **YU-WEN ZHOU** ; **LEI XU** ; **JUN-LI ZHANG** ; **CUI-YING CHEN** ; **JIAN HE**. N-Glycan Biosignatures as a Potential Diagnostic Biomarker for Early-Stage Pancreatic Cancer. *World Journal of Gastrointestinal Oncology*, 2024, vol. 16 (3), 659-69
- **WU** ; **HUANWEN** ; **SHIWEI GUO** ; **XIAODING LIU** ; **YATONG LI** ; **ZHIXI SU** ; **QIYE HE** ; **XIAOQIAN LIU et al.** Noninvasive Detection of Pancreatic Ductal Adenocarcinoma Using the Methylation Signature of Circulating Tumour DNA. *BMC Medicine*, 2022, vol. 20 (1), 458

- **XU** ; **CAIMING** ; **EUNSUNG JUN** ; **YOSHINAGA OKUGAWA** ; **YUJI TOIYAMA** ; **ERKUT BORAZAN-CI** ; **JOHN BOLTON** ; **AKINOBU TAKETOMI et al.** A Circulating Panel of circRNA Biomarkers for the Noninvasive and Early Detection of Pancreatic Ductal Adenocarcinoma. *Gastroenterology*, 2024, vol. 166 (1), 178-190

- **YU** ; **JINGRU** ; **ALEXANDER PLONER** ; **MAXIMI-LIAN KORDES** ; **MATTHIAS LÖHR** ; **MAGNUS NILSSON** ; **MARIA EVANGELINA LOPEZ DE MATURANA** ; **LIDIA ESTUDILLO et al.** Plasma Protein Biomarkers for Early Detection of Pancreatic Ductal Adenocarcinoma. *International Journal of Cancer*, 2021, vol. 148 (8), 2048-58